# EUROPEAN PATENT APPLICATION

(11) **EP 1 607 092 A1**
(43) Date of publication of application: **21.12.2005**
(21) Application number: 04720973.9
(22) Date of filing: 16.03.2004
(51) Int. Cl.: A61K 31/4188, A61K 47/38, A61K 47/34, A61K 47/32, A61K 47/36, A61K 47/12, A61K 47/14, A61P 35/00

(54) **RELEASE CONTROL COMPOSITIONS**

(30) Priority: 17.03.2003 JP 2003072855
(71) Applicant: Takeda Pharmaceutical Company Limited, Osaka-shi, Osaka 541-0045 (JP)
(72) Inventor: YANAI, Shigeo c/o Takeda Pharmaceutical Comp. Ltd., Osaka-shi, Osaka 532-8686 (JP); YAMAMOTO, K. Takeda Pharmaceutical Comp. Ltd., Osaka-shi, Osaka 532-8686 (JP)
(74) Representative: Rickard, Timothy Mark Adrian
(86) International application number: PCT/JP2004/003488
(87) International publication number: WO 2004/082679

(57) **Abstract**

A controlled release composition for oral administration, which comprises a physiologically active substance which is a compound represented by the formula: 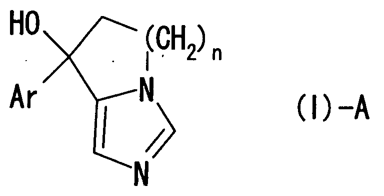 wherein n is an integer of 1 to 3, and Ar is an aromatic ring which may be substituted,
or a salt thereof, and a hydrophilic polymer, or a controlled release composition for oral administration, wherein a core containing the physiologically active substance is coated with a coating layer containing a polymer, is provided.

## Description

### Technical Field

The present invention relates to a controlled release composition. More specifically, the invention relates to a controlled release composition for oral administration comprising a steroid C_{17,20}-lyase inhibiting substance which exhibits high water solubility under acidic conditions and a hydrophilic polymer, or a controlled release composition for oral administration wherein a core containing the steroid C_{17,20}-lyase inhibiting substance is coated with a coating layer containing a polymer.

### Background Art

Preparations for oral administration such as tablets, capsules and granules can be easily administered and are safe as compared with injections or the like, thus being the most frequently used dosage forms in the medical field. In recent years, under the purpose of improving a patient's QOL (Quality of Life) and providing medical care in an energy-saving and economic way, preparations for oral administration which maintain the effect for a long time with once or twice a day administration have been developed.

Since a drug having high water solubility under acidic conditions is such that when formulated into a general rapid release preparation, it is rapidly dissolved out in the stomach and absorbed, the duration of the effective blood concentration is short. Further, there are problems such as expression of toxicity due to a sharp rise in the initial blood concentration of the drug. Therefore, it is strongly desirable to develop a controlled release preparation which is imparted with, in particular, the property of sustained release or non-release in the stomach. However, in many cases, the in vivo pharmacokinetics in blood may not be exhibited in the same way as expected from an in vitro dissolution test, and there still exist a large number of drugs which have not yet been formulated into effective controlled release preparations for oral administration.

Thus, it is an object of the invention to provide a controlled release composition for oral administration which comprises a steroid C_{17,20}-lyase inhibiting substance having high water solubility under acidic conditions as a physiologically active substance and which has remarkably improved sustainability of an effective blood concentration of the physiologically active substance.

### Disclosure of the Invention

The present inventors have conducted extensive investigations in order to achieve the above-described object and as a result, have found that a physiologically active substance (for example, a steroid C_{17,20}-lyase inhibiting substance, etc.) is included in a matrix base comprising a hydrophilic polymer, or a core containing the physiologically active substance is coated with a coating layer containing a polymer, thus the maximum blood concentration of the physiologically active substance can be significantly lowered as compared with a rapid release preparation, and thereafter, sustained drug release over a long time can be realized. They also have succeeded in improving the sustainability of the effective blood concentration of the steroid C_{17,20}-lyase inhibiting substance by making a core containing the substance into a granule coated with a coating layer containing a polymer exhibiting pH-dependent water solubility. The inventors have conducted further research based on these findings, and then have completed the invention.

That is, the present invention provides:
[1] A controlled release composition for oral administration, which comprises a physiologically active substance which is a compound represented by the formula: 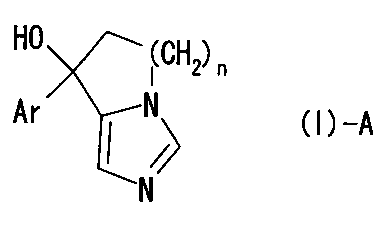 wherein n is an integer of 1 to 3, and Ar is an aromatic ring which may be substituted,
   or a salt thereof, and a hydrophilic polymer;
[2] A controlled release composition for oral administration, wherein a core containing a physiologically active substance which is a compound represented by the formula: 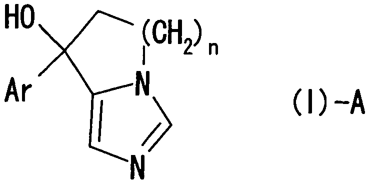 wherein n is an integer of 1 to 3, and Ar is an aromatic ring which may be substituted, or a salt thereof is coated with a coating layer containing a polymer;
[3] The controlled release composition according to the above [1] or [2], wherein the solubility (37°C) of the physiologically active substance with respect to 1^{st} fluid for the disintegration test in the Japanese Pharmacopoeia is about 0.1 mg/mL or more;
[4] The controlled release composition according to the above [1], which has the following dissolution characteristics:
   1) in Method 2 of the dissolution test in the Japanese Pharmacopoeia (paddle method, rotation speed of paddle: 50 rpm, 37°C) using 900 mL of 1^{st} fluid for the disintegration test in the Japanese Pharmacopoeia, the dissolution rate of the physiologically active substance from the controlled release composition at 15 minutes after initiation of the test is less than 40%, and
   2) in Method 2 of the dissolution test in the Japanese Pharmacopoeia (paddle method, rotation speed of paddle: 50 rpm, 37°C) using 900 mL of 2^{nd} fluid for the disintegration test in the Japanese Pharmacopoeia, the dissolution rate of the physiologically active substance from the controlled release composition at 24 hours after initiation of the test is 40% or more;
[5] A controlled release composition for oral administration, which comprises
   (1) a physiologically active substance which is a compound represented by the formula: 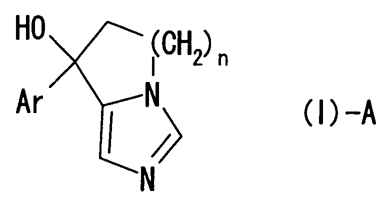 wherein n is an integer of 1 to 3, and Ar is an aromatic ring which may be substituted, or a salt thereof,
   (2) a hydrophilic polymer selected from hydroxypropylcellulose, hydroxypropylmethylcellulose, methylcellulose, polyethylene oxide, sodium carboxymethylcellulose and low-substituted hydroxypropylcellulose, and
   (3) a lubricant selected from magnesium stearate, calcium stearate, talc, light anhydrous silicic acid, colloidal silica, synthetic aluminum silicate and magnesium aluminometasilicate;
[6] The controlled release composition according to the above [2], which has the following dissolution characteristics:
   1) in Method 2 of the dissolution test in the Japanese Pharmacopoeia (paddle method, rotation speed of paddle: 50 rpm, 37°C) using 900 mL of 1^{st} fluid for the disintegration test in the Japanese Pharmacopoeia, the dissolution rate of the physiologically active substance from the controlled release composition at 15 minutes after initiation of the test is less than 10%, and
   2) in Method 2 of the dissolution test in the Japanese Pharmacopoeia (paddle method, rotation speed of paddle: 50 rpm, 37°C) using 900 mL of 2^{nd} fluid for the disintegration test in the Japanese Pharmacopoeia, the dissolution rate of the physiologically active substance from the controlled release composition at 24 hours after initiation of the test is 40% or more;
[7] A controlled release composition for oral administration, wherein
   (A) a core containing (1) a physiologically active substance which is a compound represented by the formula: 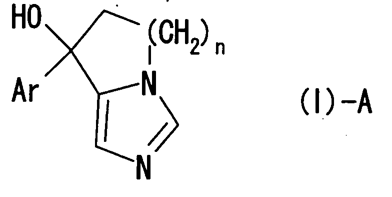 wherein n is an integer of 1 to 3, and Ar is an aromatic ring which may be substituted, or a salt thereof, and (2) a hydrophilic polymer selected from hydroxypropylcellulose, hydroxypropylmethylcellulose, methylcellulose, polyethylene oxide, sodium carboxymethylcellulose and low-substituted hydroxypropylcellulose, is coated with
   (B) a coating layer containing (1) a enteric coating agent selected from cellulose acetate phthalate, hydroxypropylmethylcellulose phthalate, hydroxymethylcellulose acetate succinate and methacrylic acid copolymers, (2) a lubricant selected from magnesium stearate, calcium stearate, talc, light anhydrous silicic acid, colloidal silica, synthetic aluminum silicate and magnesium aluminometasilicate, and (3) a plasticizer selected from acetyl tributyl citrate, acetyl triethyl citrate, castor oil, diacetylated monoglyceride, dibutyl sebacate, diethyl phthalate, glycerin, mono- and diacetylated monoglyceride, polyethylene glycol, propylene glycol, triacetin and triethyl citrate;
[8] The controlled release composition according to the above [2], wherein the release property of the physiologically active substance in the absence of the coating layer is of rapid release;
[9] The controlled release composition according to the above [2], wherein the core is a controlled release matrix which further comprises a hydrophilic polymer;
[10] The controlled release composition according to the above [1] or [9], wherein the content of the hydrophilic polymers is about 3% to about 95% by weight;
[11] The controlled release composition according to the above [2], wherein the polymer in the coating layer exhibits pH-dependent or delayed-dissolution type water solubility;
[12] The controlled release composition according to the above [2], wherein the polymer in the coating layer is insoluble or sparingly soluble in water;
[13] A controlled release composition, wherein the controlled release composition according to the above [1] or [2] is coated with a coating layer which contains a physiologically active substance which is identical with or different from the physiologically active substance contained in the above-mentioned controlled release composition, and the release property of the physiologically active substance being of rapid release;
[14] The controlled release composition according to the above [1] or [2], which is used for prevention or treatment of prostate cancer or breast cancer;
[15] A composition which comprises the controlled release composition according to the above [1] or [2], combined with at least one other controlled release composition wherein a release rate of a physiologically active substance is different from that of the above-mentioned controlled release composition;
[16] The composition according to the above [15], wherein the other controlled release composition contains a physiologically active substance whose solubility (37°C) with respect to 1^{st} fluid for the disintegration test in the Japanese Pharmacopoeia is about 0.1 mg/mL or more;
[17] The composition according to the above [16], wherein the physiologically active substance in the other controlled release composition is a compound represented by the formula: 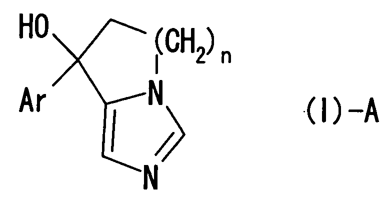 wherein n is an integer of 1 to 3, and Ar is an aromatic ring which may be substituted,
   or a salt thereof;
[18] The composition according to the above [17], which has the following dissolution characteristics:
   1) in Method 2 of the dissolution test in the Japanese Pharmacopoeia (paddle method, rotation speed of paddle: 50 rpm, 37°C) using 900 mL of 1^{st} fluid for the disintegration test in the Japanese Pharmacopoeia, the dissolution rate of the physiologically active substance from the controlled release composition at 15 minutes after initiation of the test is less than 10%, and
   2) in Method 2 of the dissolution test in the Japanese Pharmacopoeia (paddle method, rotation speed of paddle: 50 rpm, 37°C) using 900 mL of 2^{nd} fluid for the disintegration test in the Japanese Pharmacopoeia, the dissolution rate of the physiologically active substance from the controlled release composition at 24 hours after initiation of the test is 20% or more;
[19] The composition according to the above [15], wherein the release property of the physiologically active substance in the other controlled release composition is of rapid release;
[20] The composition according to the above [15], wherein the other controlled release composition is prepared by coating a core containing a physiologically active substance with a coating layer containing a polymer which exhibits pH-dependent or delayed-dissolution type water solubility;
[21] The composition according to the above [15], which is used for prevention or treatment of prostate cancer or breast cancer;
[22] A controlled release composition for oral administration, which comprises
   (1) (+)-6-(7-hydroxy-6,7-dihydro-5H-pyrrolo[1,2-c]imidazol-7-yl)-N-methyl-2-naphthamide or a salt thereof,
   (2) a hydrophilic polymer selected from hydroxypropylcellulose, hydroxypropylmethylcellulose, methylcellulose, polyethylene oxide, sodium carboxymethylcellulose and low-substituted hydroxypropylcellulose,
   (3) a disintegrant selected from lactose, sucrose, starch, carboxymethylcellulose, calcium carboxymethylcellulose, sodium croscarmellose, sodium carboxymethyl starch, light anhydrous silicic acid and low-substituted hydroxypropylcellulose,
   (4) a lubricant selected from magnesium stearate, calcium stearate, talc, light anhydrous silicic acid, colloidal silica, synthetic aluminum silicate and magnesium aluminometasilicate,
   (5) an enteric coating agent selected from cellulose acetate phthalate, hydroxypropylmethylcellulose phthalate, hydroxymethylcellulose acetate succinate and methacrylic acid copolymers,
   (6) a binder selected from α-starch, sugar, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose and polyvinylpyrrolidone, and
   (7) a plasticizer selected from acetyl tributyl citrate, acetyl triethyl citrate, castor oil, diacetylated monoglyceride, dibutyl sebacate, diethyl phthalate, glycerin, mono- and diacetylated monoglyceride, polyethylene glycol, propylene glycol, triacetin and triethyl citrate; and
[23] A controlled release composition for oral administration, wherein
   (A) a core containing (1) (+)-6-(7-hydroxy-6,7-dihydro-5H-pyrrolo[1,2-c]imidazol-7-yl)-N-methyl-2-naphthamide or a salt thereof, and (2) a hydrophilic polymer selected from hydroxypropylcellulose, hydroxypropylmethylcellulose, methylcellulose, polyethylene oxide, sodium carboxymethylcellulose and low-substituted hydroxypropylcellulose, is coated with
   (B) a coating layer containing (1) a enteric coating agent selected from cellulose acetate phthalate, hydroxypropylmethylcellulose phthalate, hydroxymethylcellulose acetate succinate and methacrylic acid copolymers, (2) a lubricant selected from magnesium stearate, calcium stearate, talc, light anhydrous silicic acid, colloidal silica, synthetic aluminum silicate and magnesium aluminometasilicate, and (3) a plasticizer selected from acetyl tributyl citrate, acetyl triethyl citrate, castor oil, diacetylated monoglyceride, dibutyl sebacate, diethyl phthalate, glycerin, mono- and diacetylated monoglyceride, polyethylene glycol, propylene glycol, triacetin and triethyl citrate.

### Brief Description of the Drawings

Fig. 1 is a graph showing the change with time of the plasma concentration of the physiologically active substance released from the film-coated controlled release composition of the invention and from rapid release tablets having no coating layer, after oral administration thereof to dogs.
Fig. 2 is a graph showing the change with time of the plasma concentration of the physiologically active substance released from three different film-coated controlled release compositions of the invention, each of which has a core containing different hydrophilic polymers, after oral administration thereof to dogs.
Fig. 3 is a graph showing the change with time of the plasma concentration of the physiologically active substance released from the mixed controlled release composition of the invention, after oral administration thereof to dogs.
Fig. 4 is a graph showing the change with time of the plasma concentration of the physiologically active substance released from the mixed controlled release composition of the invention, after oral administration thereof to monkeys.
Fig. 5 is a graph showing the change with time of the plasma concentration of the physiologically active substance released from the mixed controlled release composition of the invention, after oral administration thereof to dogs.
Fig. 6 is a graph showing the change with time of the plasma concentration of the physiologically active substance released from the controlled release composition of the invention, after oral administration thereof to monkeys.

### Best Mode for Carrying Out the Invention

The present invention provides a controlled release composition for oral administration which comprises a compound represented by the formula: 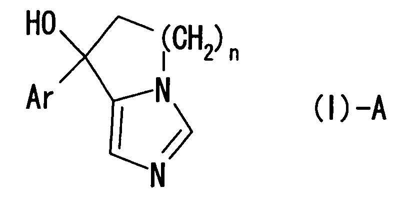 wherein n is an integer of 1 to 3, and Ar is an aromatic ring which may be substituted, or a salt thereof as a physiologically active substance, and a hydrophilic polymer.

The "controlled release composition for oral administration" as used herein means a composition for which the release profile after oral administration of the physiologically active substance contained in the composition is controlled in a manner different from the release profile shown in administration of the physiologically active substance per se, and includes any of those compositions for which the release rate is controlled to be lower (sustained release), compositions for which the release rate is controlled to be higher (accelerated dissolution), and compositions for which the time for initiation of release is controlled (delayed dissolution), further including those compositions for which two or more of these controlling fashions are combined. The controlled release composition for oral administration of the invention (hereinafter, may be simply referred to as "controlled release composition") also includes those compositions for which release is not necessarily controlled over the entire process of dissolution, and release is controlled in a part of the process.

Preferred examples of the compound represented by formula (I)-A include the following compounds:
[1] A compound wherein Ar is a monocyclic or bicyclic fused aromatic ring which may be substituted;
[2] A compound wherein Ar is an aromatic ring which may be substituted and which consists of 5 to 10 atoms including 0 to 4 heteroatoms as a ring-constituting atom, bonded via carbon atoms;
[3] A compound wherein Ar is a group represented by the formula: 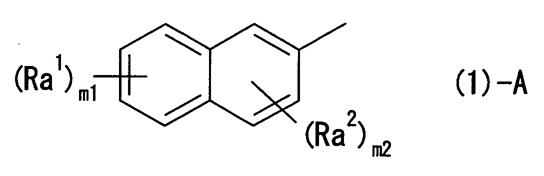 wherein m1 is an integer of 1 to 4, m2 is an integer of 0 to 3, and Ra¹ and Ra² are identical with or different from each other and are each a hydrogen atom, a hydroxyl group which may be substituted, a thiol group which may be substituted, an amino group which may be substituted, an acyl group, a halogen atom, or a hydrocarbon group which may be substituted,
   a group represented by the formula: 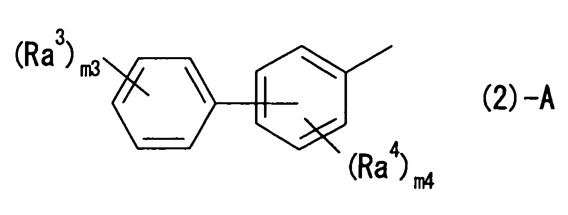 wherein m3 is an integer of 1 to 5, m4 is an integer of 0 to 4, and Ra³ and Ra⁴ are identical with or different from each other and are each a hydrogen atom, a hydroxyl group which may be substituted, a thiol group which may be substituted, an amino group which may be substituted, an acyl group, a halogen atom, or a hydrocarbon group which may be substituted, or
   a group represented by the formula: 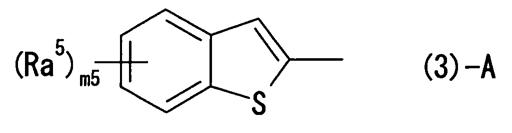 wherein m5 is an integer of 1 to 4, and Ra⁵ is a hydrogen atom, a hydroxyl group which may be substituted, a thiol group which may be substituted, an amino group which may be substituted, an acyl group, a halogen atom, or a hydrocarbon group which may be substituted;
[4] A compound wherein Ar is a group represented by the formula: 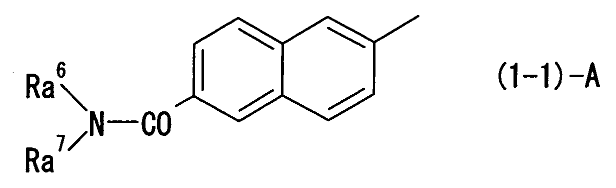 wherein Ra⁶ and Ra⁷ are identical with or different from each other and are each a hydrogen atom or a lower alkyl group, or
   a group represented by the formula: 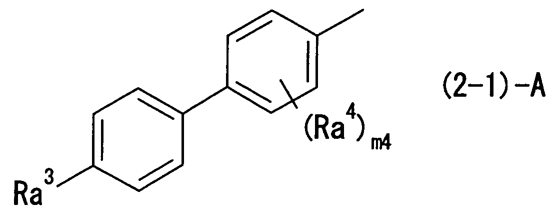 wherein m4 is an integer of 0 to 4, and Ra³ and Ra⁹ are identical with or different from each other and are each a hydrogen atom, a hydroxyl group which may be substituted, a thiol group which may be substituted, an amino group which may be substituted, an acyl group, a halogen atom, or a hydrocarbon group which may be substituted;
[5] A compound wherein Ar is a group represented by the formula: 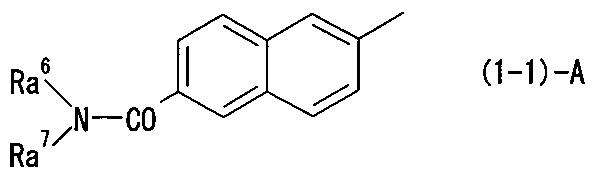 wherein Ra⁶ and Ra⁷ are identical with or different from each other and are each a hydrogen atom or a lower alkyl group;
[6] A compound which is an enantiomer whose configuration is (S); and
[7] A compound which is an enantiomer whose configuration is (R).

The definition of each symbol in each formula is as follows:
n is an integer of 1 to 3, and preferably 1.
m1 is an integer of 1 to 4, preferably 1 or 2, and particularly preferably 1.
m2 is an integer of 0 to 3, preferably 0 or 1, and particularly preferably 0.
m3 is an integer of 1 to 5, preferably 1 to 3, and particularly preferably 1.
m4 is an integer of 0 to 4, preferably 0 or 1, and particularly preferably 0.
m5 is an integer of 1 to 4, preferably 1 or 2, and particularly preferably 1.
m6 is an integer of 0 to 3, preferably 0 or 1, and particularly preferably 0.

The hydroxyl group which may be substituted represented by Ra¹, Ra², Ra³, Ra⁹ and Ra⁵ may be exemplified by, in addition to an unsubstituted hydroxyl group, a lower alkoxy (for example, C₁₋₄ alkoxy such as methoxy, ethoxy and propoxy), a lower alkanoyloxy (for example, C₁₋₄ alkanoyloxy such as acetyloxy and propionyloxy), a carbamoyloxy which may be substituted (in addition to an unsubstituted carbamoyloxy, a carbamoyloxy substituted with one or two C₁₋₄ alkyls, such as, for example, methylcarbamoyloxy, ethylcarbamoyloxy, dimethylcarbamoyloxy, diethylcarbamoyloxy and ethylmethylcarbamoyloxy), and the like.

The thiol which may be substituted represented by Ra¹, Ra², Ra³, Ra⁴ and Ra⁵ may be exemplified by, in addition to an unsubstituted thiol, a lower alkylthio (for example, C₁₋₄ alkylthio such as methylthio, ethylthio and propylthio), a lower alkanoylthio group (for example, C₁₋₄ alkanoylthio such as acetylthio and propionylthio), and the like.

The amino which may be substituted represented by Ra¹, Ra², Ra³, Ra⁴ and Ra⁵ may be exemplified by, in addition to an unsubstituted amino, a lower alkylamino (for example, C₁₋₄ alkylamino such as methylamino, ethylamino and propylamino), a di-lower alkylamino (for example, di-C₁₋₄ alkylamino such as dimethylamino and diethylamino), C₁₋₄ alkanoylamino (for example, acetylamino, propionylamino, etc.), and the like.

The acyl represented by Ra¹, Ra², Ra³, Ra⁴ and Ra⁵ may be exemplified by alkanoyl (for example, C₁₋₆ alkanoyl such as formyl, acetyl and propionyl), alkylsulfonyl (for example, C₁₋₄ alkylsulfonyl such as methylsulfonyl and ethylsulfonyl), aroyl (for example, benzoyl, toluoyl, naphthoyl, etc.), a carbamoyl which may be substituted (for example, mono- or di-C₁₋₁₀ alkylcarbamoyl such as methylcarbamoyl, ethylcarbamoyl, dimethylcarbamoyl and diethylcarbamoyl; mono- or di-C₆₋₁₄ arylcarbamoyl such as, for example, phenylcarbamoyl and diphenylcarbamoyl; mono- or di-C₇₋₁₆ aralkylcarbamoyl such as, for example, benzylcarbamoyl and dibenzylcarbamoyl; etc.), a sulfamoyl which may be substituted (for example, mono- or di-C₁₋₁₀ alkylsulfamoyl such as methylsulfamoyl, ethylsulfamoyl, dimethylsulfamoyl and diethylsulfamoyl; mono- or di-C₆₋₁₄ arylsulfamoyl such as, for example, phenylsulfamoyl and diphenylsulfamoyl; mono- or di-C₇₋₁₆ aralkylsulfamoyl such as, for example, benzylsulfamoyl and dibenzylsulfamoyl; etc.), and the like.

The halogen represented by Ra¹, Ra², Ra³, Ra⁴ and Ra⁵ may be exemplified by fluorine, chlorine, bromine, and iodine.

The "hydrocarbon group" of the "hydrocarbon group which may be substituted" represented by Ra¹, Ra², Ra³, Ra⁴ and Ra⁵ may be exemplified by a chain hydrocarbon group, a cyclic hydrocarbon group or the like.

The chain hydrocarbon group may be exemplified by a straight-chained or branched hydrocarbon group having 1 to 10 carbon atoms or the like, and specifically by alkyl, alkenyl, alkynyl or the like. Among these, alkyl is particularly preferred. Examples of the "alkyl" include C₁₋₁₀ alkyl such as methyl, ethyl, propyl, isopropyl, butyl, isobutyl, sec-butyl, tert-butyl, pentyl, isopentyl, neopentyl, hexyl and isohexyl, and the like, and preferred is C₁₋₆ alkyl (for example, methyl, ethyl, propyl, isopropyl, butyl, sec-butyl, tert-butyl, etc.). Examples of the "alkenyl" include C₂₋₁₀ alkenyl such as vinyl, 1-propenyl, allyl, isopropenyl, 1-butenyl, 2-butenyl, 3-butenyl, isobutenyl and sec-butenyl, and the like, and preferred is C₂₋₆ alkenyl (for example, vinyl, 1-propenyl, allyl, etc.). Examples of the "alkynyl" include C₂₋₁₀ alkynyl such as ethynyl, 1-propynyl and propargyl, and the like, and preferred is C₂₋₆ alkynyl (for example, ethynyl, etc.).

The cyclic hydrocarbon group may be exemplified by a cyclic hydrocarbon group having 3 to 18 carbon atoms, and specifically by an alicyclic hydrocarbon group, an aromatic hydrocarbon group or the like.

The "alicyclic hydrocarbon group" may be exemplified by a monocyclic or fused polycyclic group having 3 to 10 carbon atoms, and specifically by cycloalkyl, cycloalkenyl and bi- or tricyclic fused rings formed therefrom with C₆₋₁₄ aryl (for example, benzene, etc.), or the like. Examples of the "cycloalkyl" include C₃₋₆ cycloalkyl such as cyclopropyl, cyclobutyl, cyclopentyl and cyclohexyl, and the like, and examples of the "cycloalkenyl" include C₃₋₆ cycloalkenyl such as cyclopropenyl, cyclobutenyl, cyclopentenyl and cyclohexenyl, and the like.

The "aromatic hydrocarbon group" may be exemplified by a monocyclic aromatic hydrocarbon group or a fused polycyclic aromatic hydrocarbon group consisting of 6 to 18 carbon atoms, or the like, and specifically by C₆₋₁₄ aryl such as phenyl, 1-naphthyl, 2-naphthyl, 2-indenyl and 2-anthryl, and the like. C₆₋₁₀ aryl (for example, phenyl, etc.) or the like is preferred.

The substituent which may be carried by the "chain hydrocarbon group" of the "hydrocarbon group which may be substituted" is not particularly limited, and examples thereof include a halogen atom, a hydroxyl group, alkoxy, acyloxy, alkylthio, acylamino, carboxyl, alkoxycarbonyl, oxo, alkylcarbonyl, cycloalkyl, aryl, an aromatic heterocyclic group and the like. These substituents are substituted on the "chain hydrocarbon group" to a chemically acceptable extent, and the number of the substituents is from 1 to 5, and preferably from 1 to 3, provided that when the number of the substituents is two or more, they may be identical with or different from each other.

The substituent which may be carried by the "cyclic hydrocarbon group" of the "hydrocarbon group which may be substituted" is not particularly limited, and examples thereof include a halogen atom, a hydroxyl group, alkoxy, acyloxy, alkylthio, alkylsulfonyl, mono- or dialkylamino, acylamino, carboxyl, alkoxycarbonyl, alkynylcarbonyl, alkyl, cycloalkyl, aryl, an aromatic heterocyclic group and the like. These substituents are substituted on the "cyclic hydrocarbon group" to a chemically acceptable extent, and the number of the substituents is from 1 to 5, and preferably from 1 to 3, provided that when the number of the substituents is two or more, they may be identical with or different from each other.

Examples of the "halogen atom" include fluorine, chlorine, bromine and iodine. Examples of the "alkoxy" include C₁₋₁₀ alkoxy such as methoxy, ethoxy, propoxy, isopropoxy, butoxy, isobutoxy, sec-butoxy, pentyloxy and hexyloxy, and the like. Examples of the "acyloxy" include formyloxy, C₁₋₁₀ alkyl-carbonyloxy (for example, acetoxy, propionyloxy, etc.) and the like. Examples of the "alkylthio" include C₁₋₁₀ alkylthio such as methylthio, ethylthio, propylthio and isopropylthio, and the like. Examples of the "alkylsulfonyl" include C₁₋₁₀ alkylsulfonyl such as methylsulfonyl, ethylsulfonyl and propylsulfonyl, and the like. Examples of the "acylamino" include formylamino, diformylamino, mono- or di-C₁₋₁₀ alkylcarbonylamino (for example, acetylamino, propionylamino, butyrylamino, diacetylamino, etc.) and the like. The "mono- or dialkylamino" is exemplified by the same lower alkylamino or di-lower alkylamino described above. Examples of the "alkoxycarbonyl" include C₁₋₁₀ alkoxycarbonyl such as methoxycarbonyl, ethoxycarbonyl, propoxycarbonyl, isopropoxycarbonyl and butoxycarbonyl, and the like. Examples of the "alkylcarbonyl" include C₁₋₁₀ alkylcarbonyl such as acetyl, propionyl, butyryl and valeryl, and the like. Examples of the "alkynylcarbonyl" include C₃₋₁₀ alkynylcarbonyl such as ethynylcarbonyl, 1-propynylcarbonyl and 2-propynylcarbonyl, and the like. Examples of the "cycloalkyl" include C₃₋₁₀ cycloalkyl such as cyclopropyl, cyclobutyl, cyclopentyl and cyclohexyl, and the like. Examples of the "aryl" include C₆₋₁₄ aryl such as phenyl, 1-naphthyl and 2-naphthyl, and the like. The "aromatic heterocyclic group" may be exemplified by monocyclic to tricyclic aromatic heterocyclic groups containing, in addition to carbon atoms, preferably 1 to 4 heteroatoms of one or two kinds selected from nitrogen, oxygen and sulfur, or the like. Specific examples thereof include thienyl, pyridyl, furyl, pyrazinyl, pyrimidinyl, imidazolyl, pyrazolyl, thiazolyl, isothiazolyl, oxazolyl, isoxazolyl, pyridazinyl, tetrazolyl, quinolyl, indolyl, isoindolyl and the like. Examples of the "alkyl" include C₁₋₁₀ alkyl such as methyl, ethyl, propyl, isopropyl, butyl, sec-butyl, tert-butyl and pentyl, and the like.

The substituent which may be carried by the above-mentioned "hydrocarbon group" may be substituted to a chemically acceptable extent having 1 to 5, and preferably 1 to 3 substituents which are further described below. Examples of such substituent include a halogen atom (for example, fluorine, chlorine and bromine), a hydroxyl group, and C₁₋₆ alkoxy (for example, methoxy, ethoxy, propoxy, isopropoxy, etc.).

The lower alkyl group represented by Ra⁶ and Ra⁷ is exemplified by a straight-chained, branched or cyclic alkyl group having 1 to 4 carbon atoms, and examples thereof include methyl, ethyl, propyl, isopropyl, butyl, isobutyl, sec-butyl, tert-butyl, cyclopropyl, cyclobutyl and the like.

The aromatic ring which may be substituted represented by Ar is exemplified by a monocyclic or bicyclic aromatic fused ring which may have 1 or more substituents, or the like. Further, an aromatic ring which may be substituted and consists of 5 to 10 atoms including 0 to 4 heteroatoms as the ring-constituting atom (here, the aromatic ring is bonded to the fused imidazole ring of the formula (I)-A via carbon atoms, not via heteroatoms) is also a suitable example of Ar.

The substituent of the aromatic ring which may be substituted represented by Ar may be exemplified by a hydroxyl group which may be substituted, a thiol which may be substituted, an amino which may be substituted, an acyl group, a halogen atom, or a hydrocarbon group which may be substituted. The "hydroxyl group which may be substituted", the "amino which may be substituted", the "acyl", the "halogen atom" and the "hydrocarbon group which may be substituted" may be exemplified by those exemplified in the above as Ra¹, Ra², Ra³, Ra⁴ and Ra⁵, respectively.

The compound represented by the formula (I)-A may be in the form of a salt, and examples of the salt include acid addition salts, for example, inorganic acid salts (for example, hydrochloride, sulfate, hydrobromide, phosphate, etc.), organic acid salts (for example, acetate, trifluoroacetate, succinate, maleate, fumarate, propionate, citrate, tartrate, lactate, oxalate, methanesulfonate, p-toluenesulfonate, etc.) and the like.

Furthermore, the compound represented by the formula (I)-A or a salt thereof may be in the form of a hydrate, and any of these fall within the scope of the invention. Hereinafter, salts and hydrates are also included into what is referred to as Compound (I)-A.

The compound represented by the formula (I)-A and a prodrug thereof have excellent effect as a medicine and particularly have excellent inhibitory activity against steroid C_{17,20}-lyase. These compounds have low toxicity and few side effects, and thus they are useful for mammals (for example, a human, a cow, a horse, a pig, a dog, a cat, a monkey, a mouse, a rat, etc., especially a human), for example, as (i) an androgen or estrogen-lowering drug [i.e., a medicine having an inhibitory action on production of androgen and subsequent production of estrogen (estrogen is synthesized from androgen as the substrate)], and (ii) a therapeutic and prophylactic drug for the diseases related to androgen or estrogen, for example, (1) primary cancer, metastasis or recurrence of malignant tumors (for example, prostate cancer, breast cancer, uterine cancer, ovarian cancer, etc.), (2) symptoms associated with those cancers (for example, pain, cachexia, etc.), and (3) various diseases such as benign prostatic hypertrophy, virilism, hypertrichosis, male pattern baldness, sexual precocity in boys, endometriosis, hysteromyoma, uterine adenomyosis, mastopathy and polycystic ovarian syndrome, and preferably prostate cancer, breast cancer and the like.

The prodrug of Compound (I)-A refers to a compound which is converted to Compound (I)-A by an in vivo reaction under the action of enzyme, gastric acid or the like. Since the prodrug exhibits steroid C_{17,20}-lyase inhibitory activity upon conversion to Compound (I)-A in vivo, it is to be included in the physiologically active substance contained in the controlled release composition of the invention.

Examples of the prodrug of Compound (I)-A include compounds resulting from acylation or alkylation of the imidazole nitrogen of Compound (I)-A (for example, compounds in which the imidazole nitrogen is in the form of dimethylaminosulfonyl, acetoxymethyl, (5-methyl-2-oxo-1,3-dioxolen-4-yl)methoxycarbonylmethyl, pivaloyloxymethyl, benzyloxymethyl, etc.); compounds resulting from acylation, alkylation, phosphorylation, sulfation or boration of the hydroxyl group of Compound (I)-A (for example, compounds in which the hydroxyl group of Compound (I)-A is in the form of acetyl, palmitoyl, propanoyl, pivaloyl, succinyl, fumaryl, alanyl, dimethylaminomethylcarbonyl or the like); and the like. These compounds can be prepared by those methods known per se in the art.

The prodrug of Compound (I)-A may exist as such or as a pharmaceutically acceptable salt. Examples of such salt include, in the case where the prodrug of Compound (I)-A has an acidic group such as carboxyl, salts formed with inorganic bases (for example, alkali metals such as sodium and potassium; alkaline earth metals such as calcium and magnesium; transition metals such as zinc, iron and copper; etc.), organic bases (for example, organic amines such as trimethylamine, triethylamine, pyridine, picoline, ethanolamine, diethanolamine, triethanolamine, dicyclohexylamine, N,N'-dibenzylethylenediamine, tromethamine [tris(hydroxymethyl)methylamine] and tert-butylamine; basic amino acids such as arginine, lysine or ornithine; etc.), and the like.

In the case where the prodrug of Compound (I)-A has a basic group such as amino, examples of the salt include salts formed with inorganic acids or organic acids (for example, hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.), acidic amino acids such as aspartic acid and glutamic acid, and the like.

The prodrug of Compound (I)-A may be also either a hydrate or a non-hydrate.

Compound (I)-A has one or more chiral carbon atoms in the molecule, and both (R) configuration and (S) configuration with respect to these chiral carbon atoms are included in the invention.

Compound (I)-A is preferably a compound in which the absolute configuration of the hydroxyl group-bonded carbon atom is (S).

Among compounds represented by the formula (I)-A, inter alia, (±)-7-(5-methoxybenzo[b]thiophen-2-yl)-6,7-dihydro-5H-pyrrolo[1,2-c]imidazol-7-ol, (±)-7-(5-fluorobenzo[b]thiophen-2-yl)-6,7-dihydro-5H-pyrrolo[1,2-c]imidazol-7-ol, (±)-7-(4'-fluoro[1,1'-biphenyl]-3-yl)-6,7-dihydro-5H-pyrrolo[1,2-c]imidazol-7-ol, (±)-7-(4'-fluoro[1,1'-biphenyl]-4-yl)-6,7-dihydro-5H-pyrrolo[1,2-c]imidazol-7-ol, (±)-6-(7-hydroxy-6,7-dihydro-5H-pyrrolo[1,2-c]imidazol-7-yl)-N-methyl-2-naphthamide, (±)-N-cyclopropyl-6-(7-hydroxy-6,7-dihydro-5H-pyrrolo[1,2-c]imidazol-7-yl)-2-naphthamide, (±)-N-ethyl-6-(7-hydroxy-6,7-dihydro-5H-pyrrolo[1,2-c]imidazol-7-yl)-2-naphthamide, (±)-N-cyclobutyl-6-(7-hydroxy-6,7-dihydro-5H-pyrrolo[1,2-c]imidazol-7-yl)-2-naphthamide, (±)-6-(7-hydroxy-6,7-dihydro-5H-pyrrolo[1,2-c]imidazol-7-yl)-N-isopropyl-2-naphthamide, (±)-6-(7-hydroxy-6,7-dihydro-5H-pyrrolo[1,2-c]imidazol-7-yl)-2-naphthamide, (+)-7-(5-methoxybenzo[b]thiophen-2-yl)-6,7-dihydro-5H-pyrrolo[1,2-c]imidazol-7-ol, (+)-7-(5-fluorobenzo[b]thiophen-2-yl)-6,7-dihydro-5H-pyrrolo[1,2-c]imidazol-7-ol, (+)-7-(4'-fluoro[1,1'-biphenyl]-3-yl)-6,7-dihydro-5H-pyrrolo[1,2-c]imidazol-7-ol, (+)-7-(4'-fluoro[1,1'-biphenyl]-4-yl)-6,7-dihydro-5H-pyrrolo[1,2-c]imidazol-7-ol, (+)-6-(7-hydroxy-6,7-dihydro-5H-pyrrolo[1,2-c]imidazol-7-yl)-N-methyl-2-naphthamide, (+)-N-cyclopropyl-6-(7-hydroxy-6,7-dihydro-5H-pyrrolo[1,2-c]imidazol-7-yl)-2-naphthamide, (+)-N-ethyl-6-(7-hydroxy-6,7-dihydro-5H-pyrrolo[1,2-c]imidazol-7-yl)-2-naphthamide, (+)-N-cyclobutyl-6-(7-hydroxy-6,7-dihydro-5H-pyrrolo[1,2-c]imidazol-7-yl)-2-naphthamide, (+)-6-(7-hydroxy-6,7-dihydro-5H-pyrrolo[1,2-c]imidazol-7-yl)-N-isopropyl-2-naphthamide, (+)-6-(7-hydroxy-6,7-dihydro-5H-pyrrolo[1,2-c]imidazol-7-yl)-2-naphthamide, (-)-7-(5-methoxybenzo[b]thiophen-2-yl)-6,7-dihydro-5H-pyrrolo[1,2-c]imidazol-7-ol, (-)-7-(5-fluorobenzo[b]thiophen-2-yl)-6,7-dihydro-5H-pyrrolo[1,2-c]imidazol-7-ol, (-)-7-(4'-fluoro[1,1'-biphenyl]-3-yl)-6,7-dihydro-5H-pyrrolo[1,2-c]imidazol-7-ol, (-)-7-(4'-fluoro[1,1'-biphenyl]-4-yl)-6,7-dihydro-5H-pyrrolo[1,2-c]imidazol-7-ol, (-)-6-(7-hydroxy-6,7-dihydro-5H-pyrrolo[1,2-c]imidazol-7-yl)-N-methyl-2-naphthamide, (-)-N-cyclopropyl-6-(7-hydroxy-6,7-dihydro-5H-pyrrolo[1,2-c]imidazol-7-yl)-2-naphthamide, (-)-N-ethyl-6-(7-hydroxy-6,7-dihydro-5H-pyrrolo[1,2-c]imidazol-7-yl)-2-naphthamide, (-)-N-cyclobutyl-6-(7-hydroxy-6,7-dihydro-5H-pyrrolo[1,2-c]imidazol-7-yl)-2-naphthamide, (-)-6-(7-hydroxy-6,7-dihydro-5H-pyrrolo[1,2-c]imidazol-7-yl)-N-isopropyl-2-naphthamide, (-)-6-(7-hydroxy-6,7-dihydro-5H-pyrrolo[1,2-c]imidazol-7-yl)-2-naphthamide, and the like are preferred. In particular, (+)-6-(7-hydroxy-6,7-dihydro-5H-pyrrolo[1,2-c]imidazol-7-yl)-N-methyl-2-naphthamide is preferred.

The compound represented by the formula (I)-A can be prepared by, for example, the method disclosed in WO 02/40484.

Preferably, the physiologically active substance contained in the controlled release composition of the invention exhibits high water solubility under acidic conditions, and specifically, the solubility of the substance at 37°C with respect to 1^{st} fluid for the disintegration test in the Japanese Pharmacopoeia (hereinafter, may be abbreviated as "solubility (Pharmacopoeia 1^{st} fluid, 37°C)") is about 0.1 mg/mL or more, preferably about 1 mg/mL or more, and more preferably about 5 mg/mL or more.

The "hydrophilic polymers" contained in the controlled release composition of the invention refers to polymers which become hydrogel upon absorption of water, and then allow diffusion of the physiologically active substance dispersed in the gel, or become able to control release of the physiologically active substance by dissolution of the polymer itself in water, or control release of the physiologically active substance not by dissolution but by swelling in water.

The viscosity of the hydrophilic polymer is, for example, as the viscosity of a 2 wt% aqueous solution (measurement temperature: 20°C), preferably 1 mPa·s or more, and more preferably 4 mPa·s or more. With respect to the controlled release composition of the invention, it is possible to arbitrarily control the duration of release of the physiologically active substance from the composition by adjusting the viscosity of the hydrophilic polymer which is used as the base capable of controlled release, or mixing ratio thereof, or the like.

Specific examples of the hydrophilic polymer include:
hydroxypropylcellulose such as HPC-SSL (tradename, Nippon Soda Co., Ltd.) (viscosity of 2 wt% aqueous solution at 20°C: 2.0 to 2.9 mPa·s), HPC-SL (tradename, Nippon Soda Co., Ltd.) (viscosity of 2 wt% aqueous solution at 20°C: 3.0 to 5.9 mPa·s), HPC-L (tradename, Nippon Soda Co., Ltd.) (viscosity of 2 wt% aqueous solution at 20°C: 6.0 to 10.0 mPa·s), HPC-M (tradename, Nippon Soda Co., Ltd.) (viscosity of 2 wt% aqueous solution at 20°C: 150 to 400 mPa·s), HPC-H (tradename, Nippon Soda Co., Ltd.) (viscosity of 2 wt% aqueous solution at 20°C: 1000 to 4000 mPa·s), and the like;
hydroxypropylmethylcellulose such as TC-5E (tradename, Shin-Etsu Chemical Co., Ltd.) (viscosity of 2 wt% aqueous solution at 20°C: about 3 mPa·s), TC-5EW (tradename, Shin-Etsu Chemical Co., Ltd.) (viscosity of 2 wt% aqueous solution at 20°C: about 3 mPa·s), SB-4 (tradename, Shin-Etsu Chemical Co., Ltd.) (viscosity of 2 wt% aqueous solution at 20°C: about 4 mPa·s), TC-5MW (tradename, Shin-Etsu Chemical Co., Ltd.) (viscosity of 2 wt% aqueous solution at 20°C: about 4.5 mPa·s), TC-5R (tradename, Shin-Etsu Chemical Co., Ltd.) (viscosity of 2 wt% aqueous solution at 20°C: about 6 mPa·s), TC-5RW (tradename, Shin-Etsu Chemical Co., Ltd.) (viscosity of 2 wt% aqueous solution at 20°C: about 6 mPa·s), TC-5S (tradename, Shin-Etsu Chemical Co., Ltd.) (viscosity of 2 wt% aqueous solution at 20°C: about 15 mPa·s), Metolose 60SH-50 (tradename, Shin-Etsu Chemical Co., Ltd.) (viscosity of 2 wt% aqueous solution at 20°C: about 50 mPa·s), Metolose 65SH-50 (tradename, Shin-Etsu Chemical Co., Ltd.) (viscosity of 2 wt% aqueous solution at 20°C: about 50 mPa·s), Metolose 90SH-100 (tradename, Shin-Etsu Chemical Co., Ltd.) (viscosity of 2 wt% aqueous solution at 20°C: about 100 mPa·s), Metolose 65SH-400 (tradename, Shin-Etsu Chemical Co., Ltd.) (viscosity of 2 wt% aqueous solution at 20°C: about 400 mPa·s), Metolose 90SH-400 (tradename, Shin-Etsu Chemical Co., Ltd.) (viscosity of 2 wt% aqueous solution at 20°C: about 400 mPa·s), Metolose 65SH-1500 (tradename, Shin-Etsu Chemical Co., Ltd.) (viscosity of 2 wt% aqueous solution at 20°C: about 1500 mPa·s), Metolose 60SH-4000 (tradename, Shin-Etsu Chemical Co., Ltd.) (viscosity of 2 wt% aqueous solution at 20°C: about 4000 mPa·s), Metolose 65SH-4000 (tradename, Shin-Etsu Chemical Co., Ltd.) (viscosity of 2 wt% aqueous solution at 20°C: about 4000 mPa·s), Metolose 90SH-4000 (tradename, Shin-Etsu Chemical Co., Ltd.) (viscosity of 2 wt% aqueous solution at 20°C: about 4000 mPa·s), Metolose 90SH-30000 (tradename, Shin-Etsu Chemical Co., Ltd.) (viscosity of 2 wt% aqueous solution at 20°C: about 30000 mPa·s), and the like;
methylcellulose such as Metolose SM15 (tradename, Shin-Etsu Chemical Co., Ltd.) (viscosity of 2 wt% aqueous solution at 20°C: about 15 mPa·s), Metolose SM25 (tradename, Shin-Etsu Chemical Co., Ltd.) (viscosity of 2 wt% aqueous solution at 20°C: about 25 mPa·s), Metolose SM100 (tradename, Shin-Etsu Chemical Co., Ltd.) (viscosity of 2 wt% aqueous solution at 20°C: about 100 mPa·s), Metolose SM400 (tradename, Shin-Etsu Chemical Co., Ltd.) (viscosity of 2 wt% aqueous solution at 20°C: about 400 mPa·s), Metolose SM1500 (tradename, Shin-Etsu Chemical Co., Ltd.) (viscosity of 2 wt% aqueous solution at 20°C: about 1500 mPa·s), Metolose SM4000 (tradename, Shin-Etsu Chemical Co., Ltd.) (viscosity of 2 wt% aqueous solution at 20°C: about 4000 mPa·s), Metolose SM8000 (tradename, Shin-Etsu Chemical Co., Ltd.) (viscosity of 2 wt% aqueous solution at 20°C: about 8000 mPa·s), and the like;
polyethylene oxide such as WSR N-12K (tradename, Union Carbide Corp.) (viscosity of 2 wt% aqueous solution at 20°C: 400 to 800 mPa·s), WSR N-60K (tradename, Union Carbide Corp.) (viscosity of 2 wt% aqueous solution at 20°C: 2000 to 4000 mPa·s), WSR 301 (tradename, Union Carbide Corp.) (viscosity of 1 wt% aqueous solution at 25°C: 1500 to 4500 mPa·s), WSR Coagulant (tradename, Union Carbide Corp.) (viscosity of 1 wt% aqueous solution at 25°C: 4500 to 7500 mPa·s), WSR 303 (tradename, Union Carbide Corp.) (viscosity of 1 wt% aqueous solution at 25°C: 7500 to 10000 mPa·s), WSR 308 (tradename, Union Carbide Corp.) (viscosity of 1 wt% aqueous solution at 25°C: 10000 to 15000 mPa·s), and the like;
sodium carboxymethylcellulose such as Sanlose F-150MC (tradename, Nippon Paper Group, Inc.) (viscosity of 1 wt% aqueous solution at 25°C: 1200 to 1800 mPa·s), Sanlose F-300MC (tradename, Nippon Paper Group, Inc.) (viscosity of 1 wt% aqueous solution at 25°C: 2500 to 3000 mPa·s), Sanlose F-1000MC (tradename, Nippon Paper Group, Inc.) (viscosity of 1 wt% aqueous solution at 25°C: 8000 to 12000 mPa·s), and the like;
low-substituted hydroxypropylcellulose such as LH-11 (tradename, Shin-Etsu Chemical Co., Ltd.), LH-21 (tradename, Shin-Etsu Chemical Co., Ltd.), LH-31 (tradename, Shin-Etsu Chemical Co., Ltd.), LH-22 (tradename, Shin-Etsu Chemical Co., Ltd.), LH-32 (tradename, Shin-Etsu Chemical Co., Ltd.), LH-20 (tradename, Shin-Etsu Chemical Co., Ltd.), LH-30 (tradename, Shin-Etsu Chemical Co., Ltd.), and the like; and the like. These hydrophilic polymers may be used in a mixture of two or more kinds thereof at an appropriate ratio.

The content of the physiologically active substance in the controlled release composition of the invention varies depending on the kind of the physiologically active substance, the size of the preparation or the like, but it is, for example, 1 to 90% by weight, preferably 5 to 85% by weight, and more preferably 10 to 80% by weight.

Further, the content of the hydrophilic polymer in the controlled release composition of the invention varies depending on the content of the physiologically active substance, the size of the preparation, the kind of the hydrophilic polymer or the like, but it is, for example, 3 to 95% by weight, preferably 5 to 95% by weight, and more preferably 5 to 90% by weight.

When the above-described controlled release composition contains the above-described physiologically active substance and hydrophilic polymers at the above-described respective contents, immediate dissolution of the physiologically active substance at acidic pH (for example, pH 1 to 3), which corresponds to the dissolution in the vicinity of stomach during the early stage of oral administration, is controlled; and dissolution of the physiologically active substance at weakly acidic to weakly alkaline pH (for example, pH 5 to 8), which corresponds to the subsequent dissolution in the small intestine or less, particularly in the duodenum to ileum, is sustained for a long time.

Specifically, in the above-described controlled release composition,
1) in Method 2 of the dissolution test in the Japanese Pharmacopoeia (paddle method, rotation speed of paddle: 50 rpm, 37°C) using 900 mL of 1^{st} fluid for the disintegration test in the Japanese Pharmacopoeia, the dissolution rate of the physiologically active substance from the controlled release composition at 15 minutes after initiation of the test is less than 40%; and
2) in Method 2 of the dissolution test in the Japanese Pharmacopoeia (paddle method, rotation speed of paddle: 50 rpm, 37°C) using 900 mL of 2^{nd} fluid for the disintegration test in the Japanese Pharmacopoeia, the dissolution rate of the physiologically active substance from the controlled release composition at 24 hours after initiation of the test is 40% or more.

The dosage form of the above-described controlled release composition can be any suitable form for oral administration such as tablets, granules, powders, pellets capsules, crystals, pastes and the like. Among these, tablets, capsules, granules and the like are preferred.

The controlled release composition can be prepared by mixing the above-described physiologically active substance and hydrophilic polymers at the above-described respective contents and molding the mixture. Here, mixing and molding can be carried out according to methods that are conventionally used in the art of formulation technology. The mode of dispersion of the physiologically active substance in the molded product may be homogeneous dispersion or heterogeneous dispersion, but homogeneous dispersion is preferred.

Furthermore, during the process of mixing and/or molding, a pharmaceutically acceptable carrier may be also used. Here, the "pharmaceutically acceptable carrier" refers to various organic or inorganic carrier materials that are conventionally used as a material for preparation, for example, an excipient, a lubricant, a binder, a disintegrant and the like. Further, if necessary, additives for preparation such as an antiseptic, an antioxidant, a colorant, a sweetener and the like can be also used.

Preferred examples of the excipient include lactose, sucrose, D-mannitol, D-sorbitol, starch, α-starch, dextrin, crystalline cellulose, low-substituted hydroxypropylcellulose, sodium carboxymethylcellulose, gum arabic, dextrin, pullulan, light anhydrous silicic acid, synthetic aluminum silicate, magnesium aluminometasilicate and the like.

Preferred examples of the lubricant include magnesium stearate, calcium stearate, talc, light anhydrous silicic acid, colloidal silica, synthetic aluminum silicate, magnesium aluminometasilicate and the like.

Preferred examples of the binder include α-starch, sugar, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, polyvinylpyrrolidone and the like.

Preferred examples of the disintegrant include lactose, sucrose, starch, carboxymethylcellulose, calcium carboxymethylcellulose, sodium croscarmellose, sodium carboxymethyl starch, light anhydrous silicic acid, low-substituted hydroxypropylcellulose and the like.

Preferred examples of the antiseptic include paraoxybenzoic acid esters, chlorobutanol, benzyl alcohol, phenethyl alcohol, dehydroacetic acid, sorbic acid and the like.

Preferred examples of the antioxidant include sulfites, ascorbates and the like.

Preferred examples of the colorant include water-soluble edible tar dyes (for example, edible dyes such as Edible Red No. 2 and No. 3, Edible Yellow No. 4 and No. 5, Edible Blue No. 1 and No. 2, etc.), water-insoluble lake dyes (for example, aluminum salts of the above-mentioned water-soluble edible tar dyes), natural dyes (for example, β-carotene, chlorophyll, Bengala) and the like.

Preferred examples of the sweetener include saccharin sodium, dipotassium glycyrrhizinate, aspartame, stevia and the like.

Specifically, the above-described controlled release composition is preferably constituted of (1) a physiologically active substance which is a compound represented by the formula: 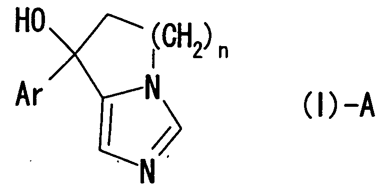 wherein n is an integer of 1 to 3, and Ar is an aromatic ring which may be substituted, or a salt thereof, (2) hydrophilic polymers selected from hydroxypropylcellulose, hydroxypropylmethylcellulose, methylcellulose, polyethylene oxide, sodium carboxymethylcellulose and low-substituted hydroxypropylcellulose, and (3) a lubricant selected from magnesium stearate, calcium stearate, talc, light anhydrous silicic acid, colloidal silica, synthetic aluminum silicate and magnesium aluminometasilicate.

When the physiologically active substance contained in the above-described controlled release composition is from weakly acidic to weakly alkaline, having relative low water solubility, and thus there is a fear that when the composition is used as a preparation for oral administration, dissolution and absorption of the physiologically active substance in the small intestine and thereafter are insufficient, a pH adjusting agent or other solubilizing aids may be added for the purpose of controlling the dissolution behavior of the composition. With the use of pH adjusting agent or the like, it is possible to reduce any change in the drug dissolution property due to the environmental pH. Further, since individual patients may have different in vivo pH values, in order to obtain uniform effect in various patients, reduction of change in the drug dissolution property due to environmental pH is significantly meaningful.

Examples of the pH adjusting agent include organic acids such as citric acid, tartaric acid, adipic acid, ascorbic acid, malic acid, fumaric acid, malonic acid, succinic acid, maleic acid, aspartic acid, glutamic acid, etc. or salts thereof (for example, sodium dihydrogen citrate, disodium citrate, calcium citrate, monosodium fumarate, monosodium succinate, sodium aspartate, magnesium aspartate, arginine glutamate, potassium glutamate, sodium glutamate, etc.), inorganic acids such as phosphoric acid, hydrochloric acid, sulfuric acid, etc. and salts thereof (for example, potassium dihydrogen phosphate, sodium dihydrogen phosphate, etc.), acidic polymers such as carboxyvinyl polymer, etc. and salts thereof. Among these, citric acid, tartaric acid, fumaric acid, ascorbic acid, aspartic acid, glutamic acid and salts thereof, and the like are preferred. In addition, examples of the other solubilizing aids include cyclodextrins such as β-cyclodextrin, maltosyl-β-cyclodextrin, etc., surfactants such as Polysorbate 80, glycerin monostearate, etc., polyethylene glycols such as Polyethylene Glycol 4000, Polyethylene Glycol 6000, etc., and the like.

The content of the pH adjusting agent or the other solubilizing aid varies depending on the kind and content of the physiologically active substance, the size of the composition or the like, but it is, for example, from 1 to 50% by weight, and preferably from 5 to 40% by weight.

When the above-described controlled release composition is in a granule form, the granules can be prepared by, for example, a centrifugal tumbling granulation in which while a binder dissolved in a suitable solvent such as water, lower alcohol (for example, methanol, ethanol, etc.) or the like is sprayed on inert carrier particles, the above-described physiologically active substance and hydrophilic polymers, or a mixture of these with an excipient, a lubricant or the like, is sprayed in small portions as a solution or a suspension, or added in powder form; pan coating, fluidized bed coating, melt granulation, extrusion and spheronization, or the like. For the inert carrier particle, for example, those prepared from sucrose, lactose, starch, crystalline cellulose or waxes can be used, and the average particle diameter of such particles is preferably about 100 µm to about 1,500 µm.

When the above-described controlled release composition is in a tablet form, the tablets can be prepared by adding hydrophilic polymers, or further the above-mentioned excipient, disintegrant, binder, lubricant or the like, to the physiologically active substance, mixing (if necessary, further kneading) and compression molding the mixture.

Since the above controlled release composition has low toxicity and few side effects, it can be orally administered safely to mammals (for example, a human, a cow, a horse, a pig, a dog, a cat, a monkey, a mouse, a rat, etc., and particularly a human), and in particular, the composition can be used as a therapeutic and prophylactic medicine for the above-mentioned various diseases, for which the prophylactic and/or therapeutic effect can be obtained by inhibiting a steroid C_{17,20}-lyase, and preferably for prostate cancer, breast cancer or the like.

Dosage of the above-described controlled release composition varies depending on the kind of physiologically active substance, the subject to be administered, the frequency of administration or the like. However, for example, the daily dose in the case of oral administration to an adult patient having solid tumor (for example, patient having prostate cancer) is, as an effective amount of the physiologically active substance, typically from about 0.001 to about 500 mg/kg of body weight, preferably from about 0.01 to about 40 mg/kg of body weight, and more preferably from about 0.1 to about 20 mg/kg of body weight.

The above-described controlled release composition can have its effect further enhanced in combination with physiologically active substances having other effects. Examples of the physiologically active substances having other effects include "sexual hormone drugs (hormone-based drugs)", "alkylating agents", "antimetabolites", "anticancerous antibiotics", "plant alkaloids", "immunotherapeutic agents", "drugs inhibiting the action of cell growth factors and their receptors" and the like (hereinafter, abbreviated as combination drugs). The combination drugs can be used as different pharmaceutical compositions, or can be used in a mixed preparation prepared by adding them to the above-described controlled release composition.

Examples of the "sexual hormone drug" include fosfestrol, diethylstilbestrol, chlorotrianisene, medroxyprogesterone acetate, megestrol acetate, chlormadinone acetate, cyproteron acetate, danazol, allylestrenol, gestrinone, mepartricin, raloxifene, ormeloxifene, levormeloxifene, antiestrogen drugs (for example, tamoxifen citrate, toremifene citrate, etc.), pill preparations, mepitiostane, testolactone, aminoglutethimide, LHRH receptor modulators [LH-RH receptor agonists (for example, goserelin acetate, buserelin acetate, leuprorelin acetate, etc.), LH-RH receptor antagonists (for example, ganirelix, cetrorelix, abarelix, etc.), droloxifene, epitiostanol, ethinylestradiol sulfonate, aromatase inhibitors (for example, fadrozole hydrochloride, anastrozole, letrozole, exemestane, vorozole, formestane, etc.), antiandrogen drugs (for example, flutamide, bicartamide, nilutamide, etc.), 5α-reductase inhibitors (for example, finasteride, epristeride, etc.), adrenocorticohormone drugs (for example, cortisol, dexamethasone, prednisolone, betamethasone, triamcinolone, etc.), androgen synthesis inhibitors (for example, abiraterone, etc.), retinoid and drugs that retard retinoid metabolism blocking agents (for example, liarozole, etc.)], and the like.

Examples of the "alkylating agent" include nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, estramustine phosphate sodium, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin and the like.

Examples of the "antimetabolite" include mercaptopurine, 6-mercaptopurine riboside, thioinosine, methotrexate, enocitabine, cytarabine, cytarabine ocfosfate, ancitabine hydrochloride, 5-FU drugs (for example, fluorouracil, tegafur, UFT, doxifluridine, carmofur, gallocitabine, emitefur, etc.), aminopterine, leucovorin calcium, tabloid, butocine, calcium folinate, calcium levofolinate, cladribine, fludarabine, gemcitabine, hydroxycarbamide, pentostatin, piritrexim, idoxuridine, mitoguazone, thiazophrine and the like.

Examples of the "anticancerous antibiotics" include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride and the like.

Examples of the "plant alkaloid" include etoposide, etoposide phosphate, vinblastine sulfate, vincristine sulfate, vindesine sulfate, teniposide, paclitaxel, vinorelbine and the like.

Examples of the "immunotherapeutic agent (BRM)" include picibanil, krestin, sizofiran, lentinan, ubenimex, interferons, interleukins, macrophage colony-stimulating factor, granulocyte colony-stimulating factor, erythropoietin, lymphotoxin, BCG vaccine, Corynebacterium parvum, levamisole, polysaccharide K, procodazole and the like.

The "cell growth factor" in the phrase "drugs inhibiting the action of cell growth factors and their receptors" may be any substance which promotes cell proliferation and may be exemplified by peptides having a molecular weight of 20,000 or less, which are factors exhibiting their action at low concentrations by binding to a receptor. Specific examples thereof include (1) EGF (epidermal growth factor) or substances having substantially identical activity therewith [for example, EGF, heregulin (HER2 ligand), etc.], (2) insulin or substances having substantially identical activity therewith [for example, insulin, IGF (insulin-like growth factor)-1, IGF-2, etc.], (3) FGF (fibroblast growth factor) or substances having substantially identical activity therewith [for example, acidic FGF, basic FGF, KGF (keratinocyte growth factor), FGF-10, etc.], (4) other cell growth factors [for example, CSF (colony stimulating factor), EPO (erythropoietin), IL-2 (interleukin-2), NGF (nerve growth factor), PDGF (platelet-derived growth factor), TGF β (transforming growth factor β), HGF (hepatocyte growth factor), VEGF (vascular endothelial growth factor), etc.], and the like.

The "receptor for cell growth factor" may be any receptor capable of binding to the above-mentioned cell growth factors, and specific examples thereof include an EGF receptor, HER2 (heregulin receptor), an insulin receptor, an IGF receptor, a FGF receptor-1 or a FGF receptor-2, and the like.

The "drug inhibiting the action of cell growth factors" may be exemplified by EGF receptor antibodies including cetuximab; antibodies against cell growth factors such as HER2 antibodies and their receptors, including Herceptin; drugs inhibiting a tyrosine kinase such as Iressa (drug inhibiting an EGF receptor tyrosine kinase), GW2016 (drug inhibiting an EGF receptor/HER2 tyrosine kinase), and the compounds described in WO98-03505A and WO01-77107A (drug inhibiting a HER2 tyrosine kinase); a ribozyme inhibiting expression of cell growth factors or their receptors; antisense drugs; or the like.

In addition to the aforementioned drugs, an L-asparaginase, aceglatone, procarbazine hydrochloride, a protoporphyrin-cobalt complex salt, mercuric hematoporphyrin-sodium, topoisomerase I inhibiting drugs (for example, irinotecan, topotecan, etc.), topoisomerase II inhibiting drugs (for example, sobuzoxane, etc.), differentiation inducers (e.g., retinoid, vitamin D, etc.), angiogenesis inhibiting drugs, α-blockers (e.g., tamsulosin hydrochloride, etc.), and the like can be also used.

The combination drugs are preferably LHRH receptor modulating drugs (LHRH modulators) [for example, LHRH receptor agonists (for example, goserelin acetate, buserelin acetate, leuprorelin acetate, etc.) or LHRH receptor antagonists (for example, ganirelix, cetrorelix, abarelix, etc.)], and can be used in combination with these, so as to remove androgen or estrogen in blood more effectively.

Dosage of the combination drugs can be appropriately selected based on the amounts that are clinically used. Further, the mixing ratio of the physiologically active substance and combination drugs in the controlled release composition of the invention can be appropriately selected in accordance with the subjects to be administered, diseases to be treated, symptoms, combinations or the like. For example, when the subject to be administered is a human, the combination drug may be used in an amount of 0.01 to 100 parts by weight with respect to 1 part by weight of the aforementioned physiologically active substance.

The invention also provides a controlled release composition for oral administration wherein a core containing the above-described compound represented by the formula (I)-A or a salt or prodrug thereof as the physiologically active substance, is coated with a coating layer containing polymers, namely, a controlled release film (hereinafter, sometimes abbreviated as the "film-coated controlled release composition"), provided that the coating layer does not contain a physiologically active substance (including substances different from the physiologically active substance contained in the core). Further, the "physiologically active substance" as used herein refers to a substance contained to achieve a desired pharmaceutical effect and does not include those substances which are incorporated as additives such as excipients.

The polymer contained in the coating layer (in the present specification, hereinafter, sometimes referred to as the "coating polymer") is a pharmaceutically acceptable polymer and is not particularly limited, as long as it can form a film structure having the function of controlling release of the physiologically active substance contained in the core. There is no particular limitation on the kind of the controlled release film as well, and examples thereof include (1) a film which has relatively large micropores as can be seen in porous films and controls release of the content through the micropores, (2) a non-porous film in a film form, which controls release of the content by diffusion through the interstices between molecular chains resulting from the motion of the film-forming polymers, (3) a film which controls release of the content as a result of dissolution or decomposition of the film, and the like. In the case of the above-mentioned (1) or (2), for example, polymers which are insoluble or sparingly soluble in water can be used as the coating polymer, and in the case of (3), for example, polymers exhibiting pH-dependent or delayed-dissolution type water solubility can be used.

The polymer exhibiting pH-dependent water solubility is preferably a polymer having an acidic dissociating group, for example, which is insoluble or sparingly soluble in an acidic (pH 1 to 3) medium such as gastric juice, and is soluble in a medium in a pH range of weakly acidic to weakly alkaline (pH 5 to 8) such as intestinal juice. Examples of such polymer exhibiting pH-dependent water solubility include cellulose acetate phthalate, hydroxypropylmethylcellulose phthalate, hydroxymethylcellulose acetate succinate, methacrylic acid copolymers (methacrylic acid-methyl acrylate copolymers, methacrylic acid-ethyl acrylate copolymers, etc., for example, Eudragit L100-55, L30D-55, L100, S100, FS (tradenames, Röhm-Pharma GmbH)) and the like, which are used as enteric coating agents. These polymers may be used in mixtures of two or more species at appropriate ratios.

Specific examples of the polymer exhibiting delayed-dissolution type water solubility include hydroxypropylcellulose (HPC) such as HPC-SSL (tradename, Nippon Soda Co., Ltd.) (viscosity of 2 wt% aqueous solution at 20°C: 2.0 to 2.9 mPa·s), HPC-SL (tradename, Nippon Soda Co., Ltd.) (viscosity of 2 wt% aqueous solution at 20°C: 3.0 to 5.9 mPa·s), HPC-L (tradename, Nippon Soda Co., Ltd.) (viscosity of 2 wt% aqueous solution at 20°C: 6.0 to 10.0 mPa·s), HPC-M (tradename, Nippon Soda Co., Ltd.) (viscosity of 2 wt% aqueous solution at 20°C: 150 to 400 mPa·s), HPC-H (tradename, Nippon Soda Co., Ltd.) (viscosity of 2 wt% aqueous solution at 20°C: 1000 to 4000 mPa·s), or the like;
hydroxypropylmethylcellulose such as TC-5E (tradename, Shin-Etsu Chemical Co., Ltd.) (viscosity of 2 wt% aqueous solution at 20°C: about 3 mPa·s), TC-5-EW (tradename, Shin-Etsu Chemical Co., Ltd.) (viscosity of 2 wt% aqueous solution at 20°C: about 3 mPa·s), SB-4 (tradename, Shin-Etsu Chemical Co., Ltd.) (viscosity of 2 wt% aqueous solution at 20°C: about 4 mPa·s), TC-5MW (tradename, Shin-Etsu Chemical Co., Ltd.) (viscosity of 2 wt% aqueous solution at 20°C: about 4.5 mPa·s), TC-5R (tradename, Shin-Etsu Chemical Co., Ltd.) (viscosity of 2 wt% aqueous solution at 20°C: about 6 mPa·s), TC-5-RW (tradename, Shin-Etsu Chemical Co., Ltd.) (viscosity of 2 wt% aqueous solution at 20°C: about 6 mPa·s), TC-5S (tradename, Shin-Etsu Chemical Co., Ltd.) (viscosity of 2 wt% aqueous solution at 20°C: about 15 mPa·s), Metolose 60SH-50 (tradename, Shin-Etsu Chemical Co., Ltd.) (viscosity of 2 wt% aqueous solution at 20°C: about 50 mPa·s), Metolose 65SH-50 (tradename, Shin-Etsu Chemical Co., Ltd.) (viscosity of 2 wt% aqueous solution at 20°C: about 50 mPa·s), Metolose 90SH-100 (tradename, Shin-Etsu Chemical Co., Ltd.) (viscosity of 2 wt% aqueous solution at 20°C: about 100 mPa·s), Metolose 65SH-400 (tradename, Shin-Etsu Chemical Co., Ltd.) (viscosity of 2 wt% aqueous solution at 20°C: about 400 mPa·s), Metolose 90SH-400 (tradename, Shin-Etsu Chemical Co., Ltd.) (viscosity of 2 wt% aqueous solution at 20°C: about 400 mPa·s), Metolose 65SH-1500 (tradename, Shin-Etsu Chemical Co., Ltd.) (viscosity of 2 wt% aqueous solution at 20°C: about 1500 mPa·s), Metolose 60SH-4000 (tradename, Shin-Etsu Chemical Co., Ltd.) (viscosity of 2 wt% aqueous solution at 20°C: about 4000 mPa·s), Metolose 65SH-4000 (tradename, Shin-Etsu Chemical Co., Ltd.) (viscosity of 2 wt% aqueous solution at 20°C: about 4000 mPa·s), Metolose 90SH-4000 (tradename, Shin-Etsu Chemical Co., Ltd.) (viscosity of 2 wt% aqueous solution at 20°C: about 4000 mPa·s), Metolose 90SH-30000 (tradename, Shin-Etsu Chemical Co., Ltd.) (viscosity of 2 wt% aqueous solution at 20°C: about 30000 mPa·s), or the like;
methylcellulose such as Metolose SM15 (tradename, Shin-Etsu Chemical Co., Ltd.) (viscosity of 2 wt% aqueous solution at 20°C: about 15 mPa·s), Metolose SM25 (tradename, Shin-Etsu Chemical Co., Ltd.) (viscosity of 2 wt% aqueous solution at 20°C: about 25 mPa·s), Metolose SM100 (tradename, Shin-Etsu Chemical Co., Ltd.) (viscosity of 2 wt% aqueous solution at 20°C: about 100 mPa·s), Metolose SM400 (tradename, Shin-Etsu Chemical Co., Ltd.) (viscosity of 2 wt% aqueous solution at 20°C: about 400 mPa·s), Metolose SM1500 (tradename, Shin-Etsu Chemical Co., Ltd.) (viscosity of 2 wt% aqueous solution at 20°C: about 1500 mPa·s), Metolose SM4000 (tradename, Shin-Etsu Chemical Co., Ltd.) (viscosity of 2 wt% aqueous solution at 20°C: about 4000 mPa·s), Metolose SM8000 (tradename, Shin-Etsu Chemical Co., Ltd.) (viscosity of 2 wt% aqueous solution at 20°C: about 8000 mPa·s), or the like;
polyethylene oxide such as WSR N-12K (tradename, Union Carbide Corp.) (viscosity of 2 wt% aqueous solution at 20°C: 400 to 800 mPa·s), WSR N-60K (tradename, Union Carbide Corp.) (viscosity of 2 wt% aqueous solution at 20°C: 2000 to 4000 mPa·s), WSR 301 (tradename, Union Carbide Corp.) (viscosity of 1 wt% aqueous solution at 25°C: 1500 to 4500 mPa·s), WSR Coagulant (tradename, Union Carbide Corp.) (viscosity of 1 wt% aqueous solution at 25°C: 4500 to 7500 mPa·s), WSR 303 (tradename, Union Carbide Corp.) (viscosity of 1 wt% aqueous solution at 25°C: 7500 to 10000 mPa·s), WSR 308 (tradename, Union Carbide Corp.) (viscosity of 1 wt% aqueous solution at 25°C: 10000 to 15000 mPa·s), or the like;
sodium carboxymethylcellulose such as Sanlose F-150MC (tradename, Nippon Paper Group, Inc.) (viscosity of 1 wt% aqueous solution at 25°C: 1200 to 1800 mPa·s), Sanlose F-300MC (tradename, Nippon Paper Group, Inc.) (viscosity of 1 wt% aqueous solution at 25°C: 2500 to 3000 mPa·s), Sanlose F-1000MC (tradename, Nippon Paper Group, Inc.) (viscosity of 1 wt% aqueous solution at 25°C: 8000 to 12000 mPa·s), or the like;
low-substituted hydroxypropylcellulose such as LH-11 (tradename, Shin-Etsu Chemical Co., Ltd.), LH-21 (tradename, Shin-Etsu Chemical Co., Ltd.), LH-31 (tradename, Shin-Etsu Chemical Co., Ltd.), LH-22 (tradename, Shin-Etsu Chemical Co., Ltd.), LH-32 (tradename, Shin-Etsu Chemical Co., Ltd.), LH-20 (tradename, Shin-Etsu Chemical Co., Ltd.), LH-30 (tradename, Shin-Etsu Chemical Co., Ltd.), or the like; and the like. Preferably, it is a polymer whose viscosity of a 2 wt% aqueous solution at 20°C or viscosity of a 1 wt% aqueous solution at 25°C is not lower than 10 mPa·s. These polymers may be used in mixtures of two or more species at appropriate ratios.

The polymer which is insoluble or sparingly soluble in water includes block polymers and copolymers. The polymer which is insoluble or sparingly soluble in water refers to a polymer whose water solubility at 37°C is less than 0.1 mg/mL. The polymer is exemplified by fat-soluble bases such as carnauba wax, hydrogenated castor oil, hydrogenated rapeseed oil and polyglycerols; cellulose esters, acrylic polymers, polyvinyl acetate, polyvinyl chloride, compositions having at least one component selected from the aforementioned polymers, or mixtures thereof. Also, it includes, for example, polyvinyl acetate, polymethyl methacrylate, poly(vinyl chloride, vinyl alcohol, vinyl acetate) (terpolymer of vinyl chloride, vinyl alcohol and vinyl acetate) or the like. Further, it includes, for example, ethylcellulose, cellulose acetate, cellulose acetate butyrate, cellulose acetate propionate, nitrocellulose, polymethyl methacrylate, poly(ethyl acrylate, methyl methacrylate), for example, Eudragit NE (tradename, Röhm-Pharma GmbH), polyethylene, polyisobutylene, poly(ethyl acrylate, methyl methacrylate, trimethylammonioethyl methacrylate chloride, for example, Eudragit RS, RL (tradenames, Röhm-Pharma GmbH), compositions having at least one component selected from the aforementioned polymers, or mixtures thereof. However, the polymer is not limited to these. Commercially available latex, pseudolatex and polymer emulsions can be also used in coating.

In a preferred embodiment of the invention, the coating polymer is a methacrylic acid-methyl acrylate copolymer or a methacrylic acid -ethyl acrylate copolymer.

Further, in another preferred embodiment of the invention, the coating polymer is poly(ethyl acrylate, methyl methacrylate, trimethylammonioethyl methacrylate chloride).

In order to adjust the softening temperature of the polymer, a plasticizer may be added to the coating polymer. The softening temperature is an important factor for controlling mechanical properties of a polymer. Suitable examples of the plasticizer include acetyl tributyl citrate, acetyltriethyl citrate, castor oil, diacetylated monoglyceride, dibutyl sebacate, diethyl phthalate, glycerin, mono- and diacetylated monoglyceride, polyethylene glycol, propylene glycol, triacetin, triethyl citrate and the like, but are not limited to these.

The core containing the physiologically active substance can be prepared according to conventional methods that are known in the art of preparation technology. The core can be in the form of a tablet, a granule, powders, a pellet, a capsule, crystals, a paste, a liquid or the like, but are not limited to these.

The release property of the physiologically active substance from the core may be rapid release in the absence of the coating layer and may be also sustained release. Here, the term "rapid release" implies that when Method 2 of the dissolution test in the Japanese Pharmacopoeia (paddle method) is carried out using 900 mL of an appropriate test fluid under the conditions such as a rotation speed of paddle of 50 rpm, the drug dissolution rate from the composition at 30 minutes after initiation of the test is 80% or more, or that when Method 1 of the dissolution test in the Japanese Pharmacopoeia (rotatory basket method) is carried out using 900 mL of an appropriate test fluid under the conditions such as a rotation speed of paddle of 75 rpm, the drug dissolution rate from the composition at 30 minutes after initiation of the test is 80% or more. The term "sustained release" implies that when Method 2 (paddle method) or Method 1 (rotatory basket method) of the dissolution test in the Japanese Pharmacopoeia is carried out under the same conditions as described above, the drug dissolution rate from the preparation at 30 minutes after initiation of the test is less than 80%. The test fluid to be used is any conventional fluid in the art of preparation technology, such as water or buffer solution.

The mechanism of release of the physiologically active substance from the core in the absence of the coating layer is not particularly limited and may be any of such mechanisms as release of the physiologically active substance from the core by passive diffusion, release of the physiologically active substance resulting from erosion of the core, release of the physiologically active substance in accordance with change in the environmental pH, release of the physiologically active substance by the internal pressure of the core when the core interior absorbs environmental moisture and swells, immediate release of the physiologically active substance upon disintegration or dissolution, and the like.

Here, the "composition releasing the physiologically active substance from the composition by passive diffusion" may be exemplified by matrix compositions using the above-described hydrophilic polymers (for example, hydroxypropylcellulose, hydroxypropylmethylcellulose, polyethylene oxide, etc.), matrix compositions using fat-soluble bases (for example, carnauba wax, hydrogenated castor oil, hydrogenated rapeseed oil, polyglycerol esters of fatty acids, etc.), matrix compositions using other bases for controlled release (for example, cellulose polymers such as ethylcellulose; aminoalkyl methacrylate copolymer-RS, ethyl acrylate-methyl methacrylate copolymer suspensions [acrylic acid-based polymers such as Eudragit NE], etc.), and the like.

The "composition releasing the physiologically active substance resulting from erosion of the preparation" may be exemplified by a capsule containing polyglycolated glyceride (for example, Gelucire 50/13 (tradename, Gattefosse SA), or the like.

The "composition releasing the physiologically active substance in accordance with change in the environmental pH" may be exemplified by matrix compositions using enteric bases (for example, acrylic acid-based polymers such as methacrylic acid copolymer L [Eudragit L (tradename, Röhm-Pharma GmbH)], methacrylic acid copolymer LD [Eudragit L-30D55 (tradename, Röhm-Pharma GmbH)], methacrylic acid copolymer S [Eudragit S (tradename, Röhm-Pharma GmbH)], etc.), and the like.

The "composition releasing the physiologically active substance by the internal pressure of the composition when the composition interior absorbs environmental moisture and swells" may be exemplified by the OROS system (tradename, ALZA Corp.) or the like.

The "composition for immediately release resulting from disintegration or dissolution (rapid release composition)" may be exemplified by a composition that can be obtained by mixing the physiologically active substance and pharmaceutically acceptable carriers and molding the mixture. Here, examples of the pharmaceutically acceptable carrier include those described above. Further, mixing and molding are carried out according to conventional methods in the art of preparation technology. The mode of dispersion of the physiologically active substance in this molding product may be homogeneous dispersion or heterogeneous dispersion, but it is preferably homogeneous dispersion.

The release mechanism of the physiologically active substance is preferably immediate release resulting from disintegration or dissolution, that is, rapid release.

The core containing the physiologically active substance can be prepared by, in addition to the above-described preparation methods, for example, centrifugal tumbling granulation in which while a polymer [for example, hydroxypropylcellulose (for example, HPC-SL, etc.), hydroxypropylmethylcellulose (for example, TC-5-RW, TC-5-EW, etc.), low-substituted hydroxypropylcellulose (for example, L-HPC-32, etc.), etc.] dispersed or dissolved in a suitable solvent such as water, lower alcohol (for example, methanol, ethanol, etc.) or the like is sprayed on inert carrier particles to be the center of the core, the physiologically active substance, or a mixture of this substance with an excipient (for example, mannitol, crystalline cellulose, etc.), a lubricant (for example, talc, light anhydrous silicic acid, etc.) or the like is sprayed in small portions as a solution or a suspension, or is added in powder form; pan coating; fluidized bed coating; melt granulation; extrusion and spheronization; or the like. For the inert carrier particle, for example, those prepared from sucrose, lactose, starch, crystalline cellulose or waxes can be used, and the average particle diameter of such particles is preferably about 50 µm to about 1,500 µm.

Alternatively, the core can be also prepared as tablets by adding to the physiologically active substance, for example, the above-described excipient, disintegrant, binder, lubricant or the like, mixing (if necessary, further kneading) and molding the mixture by extrusion.

If water solubility of the physiologically active substance is relatively low at weakly acidic to weakly alkaline conditions, a pH adjusting agent or other solubilizing aids as described above may be added for the purpose of controlling the behavior of dissolution from the composition.

When the release property of the physiologically active substance from the core in the absence of the coating layer is of sustained release, preferably, it is possible to use the matrix composition containing the aforementioned physiologically active substance and hydrophilic polymers directly as the core.

The content of the physiologically active substance in the core varies depending on the kind of the physiologically active substance, size of the core or the like, but it is, for example, from 1% to 90% by weight, preferably from 5% to 85% by weight, and more preferably from 10% to 80% by weight.

When the core is in a granule or powder form, the average particle diameter is preferably from about 50 to 2000 µm, and more preferably from about 100 to about 1400 µm.

When the core is a matrix composition for controlled release containing hydrophilic polymers, the content of the hydrophilic polymer in the core varies depending on the content of the physiologically active substance, size of the core, kind of the hydrophilic polymer or the like, but it is, for example, from 3% to 95% by weight, preferably from 5% to 95% by weight, and more preferably from 10% to 90% by weight.

The film-coated controlled release composition of the invention can be in the form of a tablet, a granule, a pellet or the like depending on the form of the core, or may be a capsule preparation containing them, but the form is not limited to these.

The film-coated controlled release composition of the invention is prepared by coating the core obtained as described above with an aqueous dispersion or non-aqueous solution of the coating polymer (hereinafter, may be referred to as "coating solution") and drying. Alternatively, the composition is also prepared by compression molding the coating polymer on the periphery of the core to form a coating layer, for example, using the same conventional methods in the art of preparation technology as in the preparation of a multilayer tablet or a core-containing tablet.

As the method for coating the core with a coating solution, for example, the method of spray coating and the like may be mentioned.

The amount of the coating layer after drying is about 0.01% to about 500% by weight, preferably about 0.1% to about 300% by weight, and more preferably about 1% to about 200% by weight, with respect to the core.

Also, the film thickness of the coating layer is about 1 µm to about 10 mm, and preferably about 5 µm to about 5 mm.

As the solvent for the coating solution, water or an organic solvent can be used individually or as a mixed solvent of the two. The mixing ratio of water and organic solvent (water/organic solvent: weight ratio) in the case of using a mixed solvent may be varied in the range of 1 to 100%. The organic solvent is not particularly limited as long as it dissolves the coating polymer, but those mentioned above are preferably used. However, water or a mixed solvent of water and organic solvent is more preferably used. At this time, if necessary, an acid such as tartaric acid, citric acid, succinic acid, fumaric acid or maleic acid may be added to the coating solution as stabilizing agent.

In the case of coating by spray coating, the operation can be carried out according to general coating methods, and specifically, it can be carried out by spray coating the core with the coating solution according to, for example, fluidized bed coating, pan coating or the like. At this time, if necessary, talc, titanium oxide, magnesium stearate, calcium stearate, light anhydrous silicic acid or the like may be added as a lubricant, and glycerin fatty acid ester, hydrogenated castor oil, triethyl citrate, cetyl alcohol, stearyl alcohol or the like may be added as a plasticizer.

After coating, if needed, an antistatic agent such as talc or light anhydrous silicic acid may be mixed as well.

In the coating solution, at least one ionic, nonionic or polymeric surfactant may be added as a stabilizing agent. Suitable examples of the surfactant include diethanolamine, fatty acids, hydroxypropylmethylcellulose, hydroxypropylcellulose, monoethanolamine, nonoxynol, octoxynol, oleic acid, Poloxamers, polyoxyethylene 50 stearate, polyoxy fatty acids, polyoxyl hydrocarbon ethers, polysorbates (for example, Polysorbate 80, etc.), povidone, fatty acid salts, sodium lauryl sulfate, sorbitan esters, trolamine and the like, but are not limited to these.

In the film-coated controlled release composition of the invention, as a core containing the above-described physiologically active substance is coated with the above-described polymer using the above-described methods, immediate dissolution of the physiologically active substance at acidic pH (for example, pH 1 to 3), which corresponds to dissolution in the vicinity of the stomach during the early stage after oral administration, is suppressed, and subsequent dissolution of the physiologically active substance at weakly acidic to weakly alkaline pH (for example, pH 5 to 8), which corresponds to dissolution in the small intestine and thereafter, especially in the duodenum to ileum, is sustained for a long time. In particular, it is possible to more strictly control the dissolution during the early stage after administration, by means of the controlled release film.

Specifically, in the film-coated controlled release composition of the invention,
1) in Method 2 of the dissolution test in the Japanese Pharmacopoeia (paddle method, rotation speed of paddle: 50 rpm, 37°C) using 900 mL of 1^{st} fluid for the disintegration test in the Japanese Pharmacopoeia, the dissolution rate of the physiologically active substance from the controlled release composition at 15 minutes after initiation of the test is less than 10%; and
2) in Method 2 of the dissolution test in the Japanese Pharmacopoeia (paddle method, rotation speed of paddle: 50 rpm, 37°C) using 900 mL of 2^{nd} fluid for the disintegration test in the Japanese Pharmacopoeia, the dissolution rate of the physiologically active substance from the controlled release composition at 24 hours after initiation of the test is 40% or more.

Furthermore, specifically the film-coated controlled release composition of the invention is a controlled release composition wherein (A) a core containing (1) a physiologically active substance which is a compound represented by the formula: 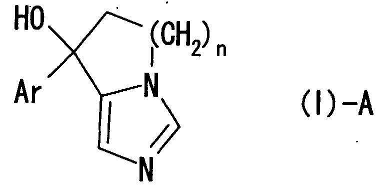 wherein n is an integer of 1 to 3, and Ar is an aromatic ring which may be substituted, or a salt thereof, and (2) hydrophilic polymers selected from hydroxypropylcellulose, hydroxypropylmethylcellulose, methylcellulose, polyethylene oxide, sodium carboxymethylcellulose and low-substituted hydroxypropylcellulose, is coated with (B) a coating layer containing (1) enteric coating agents selected from cellulose acetate phthalate, hydroxypropylmethylcellulose phthalate, hydroxymethylcellulose acetate succinate and methacrylic acid copolymer, (2) a lubricant selected from magnesium stearate, calcium stearate, talc, light anhydrous silicic acid, colloidal silica, synthetic aluminum silicate and magnesium aluminometasilicate, and (3) a plasticizer selected from acetyl tributyl citrate, acetyltriethyl citrate, castor oil, diacetylated monoglyceride, dibutyl sebacate, diethyl phthalate, glycerin, mono- and diacetylated monoglyceride, polyethylene glycol, propylene glycol, triacetin and triethyl citrate.

For the controlled release composition of the invention, a controlled release composition containing (1) (+)-6-(7-hydroxy-6,7-dihydro-5H-pyrrolo[1,2-c] imidazol-7-yl)-N-methyl-2-naphthamide or a salt thereof, (2) hydrophilic polymers selected from hydroxypropylcellulose, hydroxypropylmethylcellulose, methylcellulose, polyethylene oxide, sodium carboxymethylcellulose and low-substituted hydroxypropylcellulose, (3) a disintegrant selected from lactose, sucrose, starch, carboxymethylcellulose, calcium carboxymethylcellulose, sodium croscarmellose, sodium carboxymethylstarch, light anhydrous silicic acid and low-substituted hydroxypropylcellulose, (4) a lubricant selected from magnesium stearate, calcium stearate, talc, light anhydrous silicic acid, colloidal silica, synthetic aluminum silicate and magnesium aluminometasilicate, (5) enteric coating agents selected from cellulose acetate phthalate, hydroxypropylmethylcellulose phthalate, hydroxymethylcellulose acetate succinate and methacrylic acid copolymers, (6) a binder selected from α-starch, sugar, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose and polyvinylpyrrolidone, and (7) a plasticizer selected from acetyl tributyl citrate, acetyltriethyl citrate, castor oil, diacetylated monoglyceride, dibutyl sebacate, diethyl phthalate, glycerin, mono- and diacetylated monoglyceride, polyethylene glycol, propylene glycol, triacetin and triethyl citrate, may be mentioned as a preferred specific example. Further, a controlled release composition wherein (A) a core containing (1) (+)-6-(7-hydroxy-6,7-dihydro-5H-pyrrolo[1,2-c]imidazol-7-yl)-N-methyl-2-naphthamide or a salt thereof, and (2) hydrophilic polymers selected from hydroxypropylcellulose, hydroxypropylmethylcellulose, methylcellulose, polyethylene oxide, sodium carboxymethylcellulose and low-substituted hydroxypropylcellulose, is coated with (B) a coating agent containing (1) enteric coating agents selected from cellulose acetate phthalate, hydroxypropylmethylcellulose phthalate, hydroxymethylcellulose acetate succinate and methacrylic acid copolymers, (2) a lubricant selected from magnesium stearate, calcium stearate, talc, light anhydrous silicic acid, colloidal silica, synthetic aluminum silicate and magnesium aluminometasilicate, and (3) a plasticizer selected from acetyl tributyl citrate, acetyltriethyl citrate, castor oil, diacetylated monoglyceride, dibutyl sebacate, diethyl phthalate, glycerin, mono- and diacetylated monoglyceride, polyethylene glycol, propylene glycol, triacetin and triethyl citrate, may be also mentioned as a preferred specific example.

The film-coated controlled release composition has low toxicity and few side effects, and thus can be safely administered orally to mammals (for example, a human, a cow, a horse, a pig, a dog, a cat, a monkey, a mouse, a rat, etc., and especially a human). In particular, it can be used as a therapeutic and prophylactic medicine for the above-mentioned various diseases for which prophylactic and/or therapeutic effects can be obtained by inhibiting steroid C_{17,20}-lyase, and preferably for prostate cancer, breast cancer or the like.

Dosage of the film-coated controlled release composition varies depending on the kind of the physiologically active substance, the subject to be administered, the frequency of administration or the like, but the daily dosage in the case of, for example, oral administration to an adult patient having solid tumor (for example, prostate cancer patient) is typically about 0.001 to about 500 mg/kg of body weight, preferably about 0.1 to about 40 mg/kg of body weight, and more preferably about 0.5 to about 20 mg/kg of body weight, as an effective amount of the physiologically active substance.

The film-coated controlled release composition can have further enhanced effect when used in combination with physiologically active substances having different pharmaceutical effects. The physiologically active substance having different pharmaceutical effect may be preferably exemplified by the above-described combination drugs. The combination drugs can be used in different pharmaceutical compositions, or can be used in a mixed preparation prepared by adding them to the above-described controlled release composition.

The dosage of the combination drug can be appropriately selected on the basis of the quantities that are clinically used. Furthermore, the mixing ratio of the physiologically active substance and combination drug in the controlled release composition of the invention can be appropriately selected in accordance with the subject to be administered, disease to be treated, symptoms, combination or the like. For example, when the subject to be administered is a human, 0.01 to 100 parts by weight of a combination drug may be used with respect to 1 part by weight of the above-described physiologically active substance.

It is possible to coat the above-described controlled release composition (uncoated matrix composition) and the film-coated controlled release composition of the invention (hereinafter, both are referred to as the "controlled release composition of the invention") with a coating layer whose release property for the physiologically active substance is of rapid release. The physiologically active substance may be identical with or different from the physiologically active substance contained in the controlled release composition of the invention. If different, the physiologically active substance is preferably exemplified by the above-mentioned combination drugs or the like.

The above-described constitution allows imparting the rapid drug dissolution property during the early stage after oral administration to the controlled release composition of the invention.

In addition, the controlled release composition of the invention may be used in combination with at least one other controlled release composition, and in this case, it may be combined with a single preparation or a plurality of individual preparations. Here, the single preparation may be exemplified by a single capsule preparation encapsulating two or more controlled release compositions, a multilayer tablet having a plurality of parts for controlled release, or the like.

The physiologically active substance which is contained in the other controlled release composition so that release thereof is controlled, may be identical with or different from any of the above-mentioned compounds contained in the controlled release composition of the invention.

When the physiologically active substance contained in the other controlled release composition is different from the physiologically active substance contained in the controlled release composition of the invention, the preparation is preferably prepared as a mixed preparation aiming for additive or synergistic effect of the pharmaceutical effects, attenuation of side effects, or the like. There may be mentioned a combination of the physiologically active substances which exhibit the same pharmaceutical effect although the site of action may be identical with or different from each other; a combination in which in order to maintain the blood stability of one physiologically active substance, the other physiologically active substance inhibits or competes with the deactivating factor; a combination in which in order to avoid or delay metabolism of one physiologically active substance, the other physiologically active substance inhibits the metabolism factor; or the like.

Also preferred is a combination by which stabilization in the digestive tract, optimization of dissolution rate, improved absorption rate and the like of the physiologically active substance contained in the controlled release composition of the invention are anticipated upon release of the physiologically active substance contained in the other controlled release composition.

Preferably, the physiologically active substance contained in the other controlled release composition exhibits high water solubility under acidic conditions, and specifically, the solubility (Japanese Pharmacopoeia 1^{st} fluid, 37°C) is about 0.1 mg/mL or more, preferably about 0.5 mg/mL or more, and more preferably about 1 mg/mL or more.

More preferably, the physiologically active substance contained in the other controlled release composition is the same substance as the physiologically active substance contained in the controlled release composition of the invention, namely, the compound represented by the above-described formula (I)-A or a salt thereof.

As described above, in the controlled release composition of the invention, immediate dissolution of the physiologically active substance at acidic pH (for example, pH 1 to 3), which corresponds to dissolution in the vicinity of the stomach during the early stage after oral administration, is suppressed, and then dissolution of the physiologically active substance at weakly acidic to weakly alkaline pH (for example, pH 5 to 8), which corresponds to dissolution in the small intestine and thereafter, especially in the duodenum to ileum, is sustained for a long time. Therefore, when the other controlled release composition contains the same substance as the physiologically active substance contained in the controlled release composition of the invention, it is possible to realize the same drug dissolution and drug absorption in the digestive tract, for example, by using a rapid release composition to realize immediate dissolution in the vicinity of the gastroduodenal part and/or a timed release type controlled release composition to improve the dissolution property in the vicinity of the large intestine, as the other controlled release composition.

That is, the composition of the invention comprising the controlled release composition of the invention and at least one other controlled release composition which contains the same physiologically active substance as the physiologically active substance contained in the controlled release composition of the invention (hereinafter, also referred to the "mixed controlled release composition of the invention") has the following dissolution characteristics:
1) in Method 2 of the dissolution test in the Japanese Pharmacopoeia (paddle method, rotation speed of paddle: 50 rpm, 37°C) using 900 mL of 1^{st} fluid for the disintegration test in the Japanese Pharmacopoeia, the dissolution rate of the physiologically active substance from the controlled release composition at 15 minutes after initiation of the test is less than 10%; and
2) in Method 2 of the dissolution test in the Japanese Pharmacopoeia (paddle method, rotation speed of paddle: 50 rpm, 37°C) using 900 mL of 2^{nd} fluid for the disintegration test in the Japanese Pharmacopoeia, the dissolution rate of the physiologically active substance from the controlled release composition at 24 hours after initiation of the test is 20% or more.

Furthermore, it is desirable that the mixed controlled release composition of the invention comprises at least one other controlled release composition having a physiologically active substance whose release rate or release timing is different from the other. For example, a timed release type controlled release composition by means of a controlled release film also includes the case where the physiologically active substance in the core is dissolved out immediately after disintegration or dissolution of the film. Therefore, the aforementioned other controlled release composition also may be a composition in which the core containing a physiologically active substance is coated with a coating layer containing polymers which exhibits pH-dependent or delayed-dissolution type water solubility. For the polymer exhibiting pH-dependent or delayed-dissolution type water solubility, polymers similar to those described above can be preferably used, and for example, Eudragit L-100, S-100, FS (tradenames, Röhm-Pharma GmbH) and the like may be mentioned.

### Example

The following Reference Examples, Comparative Examples, Examples and Experimental Example further illustrate the present invention in detail, but they are not to be construed to limit the scope thereof.

Also, (+)-6-(7-hydroxy-6,7-dihydro-5H-pyrrolo[1,2-c]imidazol-7-yl)-N-methyl-2-naphthamide (hereinafter, referred to as Compound A) is used as the physiologically active substance in the following Reference Examples, Comparative Examples and Examples.

Further, for hydroxypropylcellulose, magnesium stearate, hydroxypropylmethylcellulose, polyethylene glycol 6000, red iron sesquioxide and titanium oxide, the products as specified in the 14^{th} edition of the Japanese Pharmacopoeia were used.

### Comparative Example 1

Compound A, D-mannitol, low-substituted hydroxypropylcellulose (L-HPC-11), hydroxypropylcellulose (HPC-L), magnesium stearate, hydroxypropylmethylcellulose 2910 (TC-5), polyethylene glycol 8000, titanium oxide and iron sesquioxide in the following amounts were mixed in powder form, and about 300 mg of each of the mixture was weighed and tableted into a rapid release tablet (Comparative Preparation 1) containing 100 mg of Compound A and having a diameter of about 10 mm.

| | |
|---|---|
| Compound A | 100 mg |
| D-mannitol | 133.5 mg |
| Low-substituted hydroxypropylcellulose | 42 mg |
| Hydroxypropylcellulose | 6.5 mg |
| Magnesium stearate | 3 mg |
| Hydroxypropylmethylcellulose 2910 | 10.416 mg |
| Polyethylene glycol 8000 | 2.1 mg |
| Titanium oxide | 1.4 mg |
| Iron sesquioxide | 0.084 mg |

### Example 1

About 3.2 g of Compound A, about 6.4 g of hydroxypropylmethylcellulose (TC-5MW) and about 96 mg of magnesium stearate were mixed in powder form, and about 303 mg of each of the mixture was weighed and tableted under a tableting pressure of 2.5 ton/cm², into a tablet in the 7.5R form having a diameter of 9 mm (Preparation 1).

### Example 2

About 3.2 g of Compound A, about 1.28 g of a polyethylene oxide polymer (Polyox COAG), about 5.12 g of polyethylene glycol (PEG6000) and about 96 mg of magnesium stearate were mixed in powder form, and about 303 mg of each of the mixture was weighed and tableted under a tableting pressure of 2.5 ton/cm², into a tablet in the 7.5R form having a diameter of 9 mm (Preparation 2).

### Example 3

About 3.2 g of Compound A, about 1.28 g of hydroxypropylmethylcellulose (Metolose 90SH-4000), about 5.12 g of polyethylene glycol (PEG6000) and about 96 mg of magnesium stearate were mixed in powder form, and about 303 mg of each of the mixture was weighed and tableted under a tableting pressure of 2.5 ton/cm², into a tablet in the 7.5R form having a diameter of 9 mm (Preparation 3).

### Example 4

Coating of the tablets obtained in Comparative Example 1 and Examples 1 to 3 was carried out as described below.

In about 236 g of purified water, about 4 g of polyethylene glycol 6000 as a plasticizer and about 1.4 g of Polysorbate 80 were dissolved sequentially, and then about 12 g of talc as a lubricant was uniformly dispersed therein. To the resulting solution, about 144 g of Eudragit L-30D-55 (tradename), which was a solution of a methacrylic acid copolymer, was added to yield a coating solution. Next, with respect to the tablets obtained in the above-described Preparative Examples 1 to 4, coating was carried out with about 99 g of the coating solution (about 15 g as the solids content) for about 300 g of the tablets in total, over an hour using a Hicoater (manufactured by Freund Industrial Co., Ltd., HCT-MINI), under the conditions such as the temperature of supplied air of about 70°C, rotation speed of drum of 30 rpm, spray pressure of about 0.1 MPa and coating solution injection speed of about 1.8 g/min. Weight increase in each lot of 14 tablets resulting from the coating operation was as follows, and it was confirmed that the tablet surfaces were coated with the coating ingredients:
Comparative Preparation 1: 4.198 g → 4.543 g

### (Preparation 4)

Preparation 1: 4.265 g → 4.547 g (Preparation 5)
Preparation 2: 4.213 g → 4.529 g (Preparation 6)
Preparation 3: 4.225 g → 4.530 g (Preparation 7)
Preparation numbers of the coated tablets were assigned as Preparations 4 to 7 as indicated above.

### Reference Example 1

Production of granules was carried out as described below.

In about 1128 mL of purified water, about 19.2 g of low-substituted hydroxypropylcellulose (L-HPC-32W) was added and dispersed, and subsequently about 19.2 g of hydroxypropylcellulose (HPC-SL) was added and dispersed. About 120 g of Compound A was dispersed uniformly in the resulting polymer dispersion solution to yield a coating solution. About 72 g of Celphere CP-305 as the core particle was coated with about 1126 g of the above-prepared coating solution containing Compound A, using a coating apparatus (SPIR-A-FLOW). The coating conditions were as follows: inlet temperature of about 60°C, spray pressure of about 1 kgf/cm², exhaust air scale of 100, bed pressure of about 250 mmHg, rotation speed of rotor of about 300 rpm, spray injection speed of about 2 to 8 g/min, and the spray position was disposed at the lower side. After completion of the coating operation, the granules were sieved through a 1000-µm sieve and further sieved through a 500-µm sieve, and the remaining granules on the sieve were collected and dried in vacuo at 40°C for about 24 hours. The weight of the granules obtained after drying (Reference Preparation 1) was about 170 g, and the content of Compound A therein was about 44.8% by weight of the granules.

### Example 5

Coating of the granules obtained in Reference Example 1 was carried out as described below.

In a mixed solvent comprising about 388.8 g of ethanol and about 43.2 mL of purified water, about 3 g of triethyl citrate was dissolved, and then about 30 g of Eudragit S-100, a hydrophilic polymer, was also dissolved. Into the resulting polymer solution, about 15 g of talc was uniformly dispersed to yield a coating solution. About 70 g of the Compound A-containing granules obtained in Reference Example 1 (Reference Preparation 1) was coated with about 331 g of the hydrophilic polymer-containing coating solution prepared above using a coating apparatus (SPIR-A-FLOW). The coating conditions were as follows: inlet temperature of about 40°C, spray pressure of about 1 kgf/cm², exhaust air scale of 100, bed pressure of about 250 mmHg, rotation speed of rotor of about 150 rpm, spray injection speed of about 2 to 3 g/min, and the spray position was disposed at the lower side. After completion of the coating operation, the granules were sieved through a 1000-µm sieve and further sieved through a 500-µm sieve, and the remaining granules on the sieve were collected and dried in vacuo at 40°C for about 24 hours. The weight of the granules obtained after drying (Preparation 8) was about 97 g, and the content of drug therein was about 32.8% by weight of the granules.

### Example 6

Coating of the granules obtained in Reference Example 1 was carried out as described below.

In a mixed solvent comprising about 388.8 g of ethanol and about 43.2 mL of purified water, about 3 g of triethyl citrate was dissolved, and then about 30 g of Eudragit L-100, a hydrophilic polymer, was also dissolved. Into the resulting polymer solution, about 15 g of talc was uniformly dispersed to yield a coating solution. About 70 g of the Compound A-containing granules obtained in Reference Example 1 (Reference Preparation 1) was coated with about 331 g of the hydrophilic polymer-containing coating solution prepared above using a coating apparatus (SPIR-A-FLOW). The coating conditions were as follows: inlet temperature of about 40°C, spray pressure of about 1 kgf/cm², exhaust air scale of 85 to 97, bed pressure of about 250 mmHg, rotation speed of rotor of about 150 rpm, spray injection speed of about 2 to 3 g/min, and the spray position was disposed at the lower side. After completion of the coating operation, the granules were sieved through a 1000-µm sieve and further sieved through a 500-µm sieve, and the remaining granules on the sieve were collected and dried in vacuo at 40°C for about 24 hours. The weight of the granules obtained after drying (Preparation 9) was about 101 g, and the content of drug therein was about 33.5% by weight of the granules.

### Example 7

With regard to the granules obtained in Reference Example 1, and Examples 5 and 6, Reference Preparation 1, Preparation 8 and Preparation 9 were mixed in a mixing ratio (Reference Preparation 1/Preparation 8/Preparation 9) of 1/6/3 (in terms of the amount of Compound A), so that the content of Compound A summed to 100 mg, and the mixture was encapsulated in Capsule No. 1 to give Preparation 10.

### Example 8

With regard to the granules obtained in Examples 5 and 6, Preparation 8 and Preparation 9 were mixed in a mixing ratio (Preparation 8/Prepration 9) of 3/1 (in terms of the amount of Compound A), so that the content of Compound A summed to 50 mg, and the mixture was encapsulated in Capsule No. 3 to give Preparation 11.

### Example 9

About 3 g of Compound A, about 600 mg of hydroxypropylmethylcellulose (Metolose 60SH-4000), about 3.3 g of polyethylene glycol (PEG6000), about 1.5 g of citric acid, about 600 mg of mannitol, and about 90 mg of magnesium stearate were mixed in powder form, and about 303 mg of each of the mixture was weighed and tableted under a tableting pressure of 2.5 ton/cm², into a tablet in the 7.5R form having a diameter of 9 mm to give Preparation 12.

### Example 10

Coating of the tablets obtained in Example 9 was carried out as described below.

In about 147 g of purified water, about 2.5 g of polyethylene glycol 6000 as a plasticizer and about 0.9 g of Polysorbate 80 were dissolved sequentially, and then about 7.5 g of talc as a lubricant was uniformly dispersed therein. To the resulting solution, about 90 g of Eudragit L-30D-55 (tradename), which was a solution of a methacrylic acid copolymer, was added to yield a coating solution. Next, with respect to Preparation 12, coating was carried out with about 80 g of the coating solution (about 12.2 g as the solids content) for about 300 g of the tablets in total, over about 44 minutes using a Hicoater (manufactured by Freund Industrial Co., Ltd., HCT-MINI), under the conditions such as the temperature of supplied air of about 70°C, rotation speed of drum of 30 rpm, spray pressure of about 0.1 MPa, and coating solution injection speed of about 1.8 g/min. Weight increase in 10 tablets resulting from the coating operation was from about 3.047 g to about 3.289 g, and it was confirmed that the tablet surfaces were coated with the coating ingredients. The resulting tablet was named as Preparation 13.

### Example 11

Coating of the tablets obtained in Example 9 was carried as described below.

In about 81 g of purified water, about 3.6 g of triethyl citrate as a plasticizer was dissolved, and then about 5.4 g of talc as a lubricant was uniformly dispersed therein. To the resulting solution, about 30 g of each of Eudragit RL-30D (tradename) and Eudragit RS-30D (tradename), each of which was a solution of an aminoalkyl methacrylate copolymer, was added to yield a coating solution, respectively. Next, with respect to Preparation 12, coating was carried out with about 60 g of the coating solution (about 10.8 g as the solids content) for about 300 g of the tablets in total, over about 33 minutes using a Hicoater (manufactured by Freund Industrial Co., Ltd., HCT-MINI), under the conditions such as the temperature of supplied air of about 50°C, rotation speed of drum of 30 rpm, spray pressure of about 0.1 MPa, and coating solution injection speed of about 1.8 g/min. Weight increase in 10 tablets resulting from the coating operation was from about 3.042 g to about 3.228 g, and it was confirmed that the tablet surfaces were coated with the coating ingredients. The resulting tablet was named as Preparation 14.

### Reference Example 2

Production of granules was carried out as described below.

In about 1176 mL of purified water, about 7.2 g of mannitol was dissolved, subsequently about 19.2 g of hydroxypropylcellulose (HPC-SL) was added and dispersed, and about 19.2 g of low-substituted hydroxypropylcellulose (L-HPC-32W) was added and dispersed. About 120 g of Compound A was dispersed uniformly in the resulting polymer dispersion to yield a coating solution. About 72 g of Celphere CP-507 as the core particles was coated with about 1174 g of the above-prepared coating solution containing Compound A, using a tumbling fluidized bed granulator (SPIR-A-FLOW). The coating conditions were as follows: inlet temperature of about 60°C, spray pressure of about 1 kgf/cm², exhaust air scale of 100, bed pressure of about 250 mmHg, rotation speed of rotor of about 100 rpm, spray injection speed of about 2 to 8 g/min, and the spray position being at the lower lateral side. After completion of the coating operation, the granules were sieved through a 1180-µm sieve and further sieved through a 710-µm sieve, and the remaining granules on the sieve were collected. The weight of the resulting granules (Reference Preparation 2) was about 190 g, and the content of Compound A therein was about 49% by weight of the granules.

### Reference Example 3

Production of granules was carried out as described below.

In about 607.2 mL of purified water, about 6.6 g of mannitol was dissolved, subsequently about 17.6 g of hydroxypropylmethylcellulose (TC-5-EW) was added and dispersed, and about 17.6 g of low-substituted hydroxypropylcellulose (L-HPC-32W) was added and dispersed. About 110 g of Compound A was dispersed uniformly in the resulting polymer dispersion to yield a coating solution. About 72 g of Celphere CP-507 as the core particles was coated with about 725 g of the above-prepared coating solution containing Compound A, using a tumbling fluidized bed granulator(SPIR-A-FLOW). The coating conditions were as follows: inlet temperature of about 60°C, spray pressure of about 1 kgf/cm², exhaust air scale of 100, bed pressure of about 300 mmHg, rotation speed of rotor of about 100 rpm, spray injection speed of about 8 g/min, and the spray position being at the lower lateral side. After completion of the coating operation, the granules were sieved through a 1180-µm sieve and further sieved through a 710-µm sieve, and the remaining granules on the sieve were collected. The weight of the resulting granules (Reference Preparation 3) was about 200 g.

### Reference Example 4

Production of granules was carried out as described below.

In about 619.5 mL of purified water, about 6.6 g of crystalline cellulose (PH101) was added and dispersed, subsequently about 17.6 g of hydroxypropylmethylcellulose (TC-5-EW) was added and dispersed, about 17.6 g of low-substituted hydroxypropylcellulose (L-HPC-32W) was added and dispersed, and further about 3.1 g of light anhydrous silicic acid (Aerosil) was added and dispersed. About 110 g of Compound A was dispersed uniformly in the resulting polymer dispersion to yield a coating solution. About 72 g of Celphere CP-507 as the core particle was coated with about 739 g of the above-prepared coating solution containing Compound A, using a tumbling fluidized bed granulator (SPIR-A-FLOW). The coating conditions were as follows: inlet temperature of about 60°C, spray pressure of about 1 kgf/cm², exhaust air scale of 100, bed pressure of about 300 mmHg, rotation speed of rotor of about 100 rpm, spray injection speed of about 8 g/min, and the spray position being at the lower lateral side. After completion of the coating operation, the granules were sieved through a 1180-µm sieve and further sieved through a 710-µm sieve, and the remaining granules on the sieve were collected. The weight of the resulting granules (Reference Preparation 4) was about 200 g.

### Example 12

Coating of the granules obtained in Reference Example 2 was carried out as described below.

In a mixed solvent comprising about 763.4 g of ethanol and about 84.8 mL of purified water, about 5.9 g of triethyl citrate was dissolved, and then about 44.2 g of Eudragit S-100 and about 14.7 g of Eudragit L-100 were dissolved. Into the resulting polymer solution, about 29.5 g of talc was uniformly dispersed to yield a coating solution. About 170 g of the Compound A-containing granules obtained in Reference Example 2 (Reference Preparation 2) was coated with about 900 g of the polymer-containing coating solution prepared above using atumbling fluidized bed granulator (SPIR-A-FLOW). The coating conditions were as follows: inlet temperature of about 40°C, spray pressure of about 1 kgf/cm², exhaust air scale of 100, bed pressure of about 290 mmHg, rotation speed of rotor of about 100 rpm, spray injection speed of about 4 to 5 g/min, and the spray position being at the lower lateral side. After completion of the coating operation, the granules were sieved through a 1400-µm sieve and further sieved through a 850-µm sieve, and the remaining granules on the sieve were collected. The weight of the obtained granules (Preparation 15) was about 237 g, and the content of drug therein was about 32% by weight of the granules.

### Example 13

With regard to the granules obtained in Example 12, Preparation 15 was encapsulated in Capsule No. 3 so that the content of Compound A was 50 mg, and thus Preparation 16 was obtained.

### Example 14

Coating of the granules obtained in Reference Example 3 was carried out as described below.

In a mixed solvent comprising about 285.8 g of ethanol and about 31.8 mL of purified water, about 2.2 g of triethyl citrate was dissolved, and then about 16.5 g of Eudragit S-100 and about 5.5 g of Eudragit L-100 were dissolved. Into the resulting polymer solution, about 11.0 g of talc was uniformly dispersed to yield a coating solution. About 70 g of the Compound A-containing granules obtained in Reference Example 3 (Reference Preparation 3) was coated with about 370 g of the polymer-containing coating solution prepared above using a tumbling fluidized bed granulator(SPIR-A-FLOW). The coating conditions were as follows: inlet temperature of about 40°C, spray pressure of about 1 kgf/cm², exhaust air scale of 100, bed pressure of about 300 mmHg, rotation speed of rotor of about 100 rpm, spray injection speed of about 4 to 5 g/min, and the spray position being at the lower lateral side. After completion of the coating operation, the granules were sieved through a 1400-µm sieve and further sieved through a 850-µm sieve, and the remaining granules on the sieve were collected. The weight of the obtained granules (Preparation 17) was about 85 g, and the content of drug therein was about 33% by weight of the granules.

### Example 15

Coating of the granules obtained in Reference Example 4 was carried out as described below.

In a mixed solvent comprising about 285.8 g of ethanol and about 31.8 mL of purified water, about 2.2 g of triethyl citrate was dissolved, and then about 16.5 g of Eudragit S-100 and about 5.5 g of Eudragit L-100 were dissolved. Into the resulting polymer solution, about 11.0 g of talc was uniformly dispersed to yield a coating solution. About 70 g of the Compound A-containing granules obtained in Reference Example 3 (Reference Preparation 4) was coated with about 370 g of the polymer-containing coating solution prepared above using a tumbling fluidized bed granulator (SPIR-A-FLOW). The coating conditions were as follows: inlet temperature of about 40°C, spray pressure of about 1 kgf/cm², exhaust air scale of 100, bed pressure of about 300 mmHg, rotation speed of rotor of about 100 rpm, spray injection speed of about 4 to 5 g/min, and the spray position being at the lower lateral side. After completion of the coating operation, the granules were sieved through a 1400-µm sieve and further sieved through a 850-µm sieve, and the remaining granules on the sieve were collected. The weight of the obtained granules (Preparation 18) was about 85 g, and the content of drug therein was about 32% by weight of the granules.

### Experimental Example 1

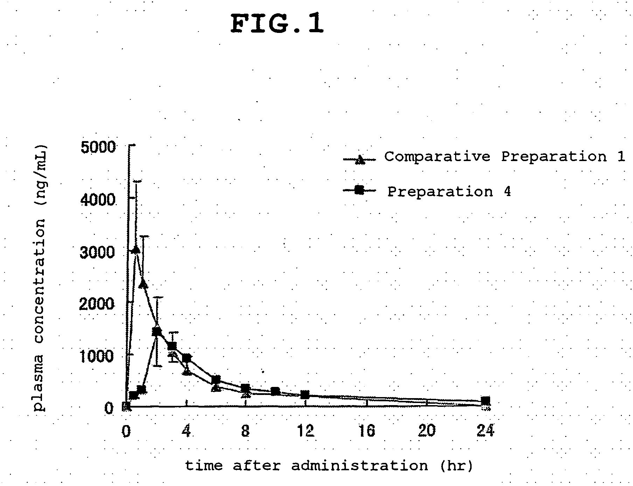

The coated tablets obtained in Example 4 (Preparation 4) was orally administered to beagle dogs (male, body weight of about 10 kg), and the change in the plasma concentration of Compound A thereafter was investigated. For comparison, the tablets obtained in Comparative Example 1 (Comparative Preparation 1) was likewise orally administered to beagle dogs, and the change in the plasma concentration of Compound A thereafter was investigated. The results are presented in Fig. 1.

As shown in Fig. 1, the blood concentration of the physiologically active ingredient (Compound A) was sustained by the controlled release composition of the invention.

### Experimental Example 2

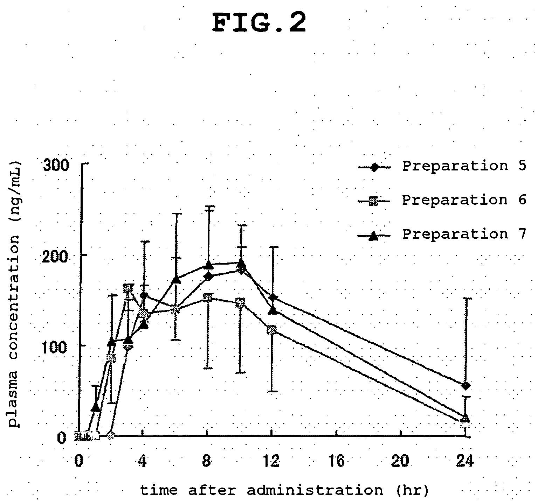

The coated tablets obtained in Example 4 (Preparations 5 to 7) were orally administered to beagle dogs (male, body weight of about 10 kg), and the change in the plasma concentration of Compound A thereafter was investigated. The results are presented in Fig. 2.

As shown in Fig. 2, the blood concentration of the physiologically active ingredient (Compound A) was sustained by the controlled release composition of the invention.

### Experimental Example 3

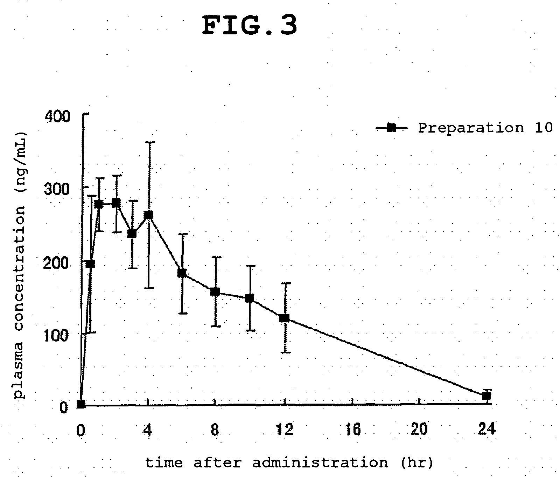

The capsules obtained in Example 7 (Preparation 10) was orally administered to beagle dogs (male, body weight of about 10 kg), and the change in the plasma concentration of Compound A thereafter was investigated. The results are presented in Fig. 3.

As shown in Fig. 3, the blood concentration of the physiologically active ingredient (Compound A) was sustained by the controlled release composition of the invention.

### Experimental Example 4

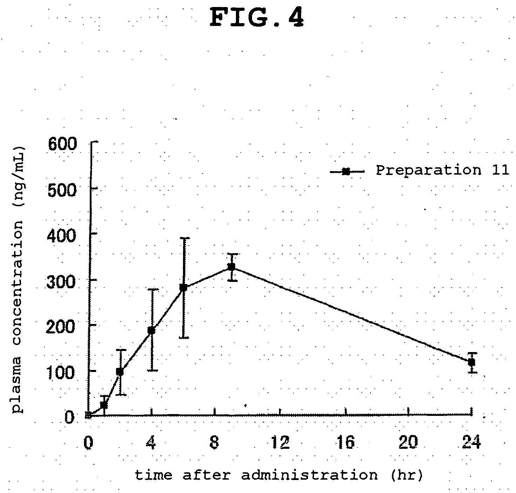

The capsules obtained in Example 8 (Preparation 11) was orally administered to a cynomolgus monkeys (male, body weight of about 4 kg), and the change in the plasma concentration of Compound A thereafter was investigated. The results are presented in Fig. 4.

As shown in Fig. 4, the blood concentration of the physiologically active ingredient (Compound A) was sustained by the controlled release composition of the invention. Experimental Example 5

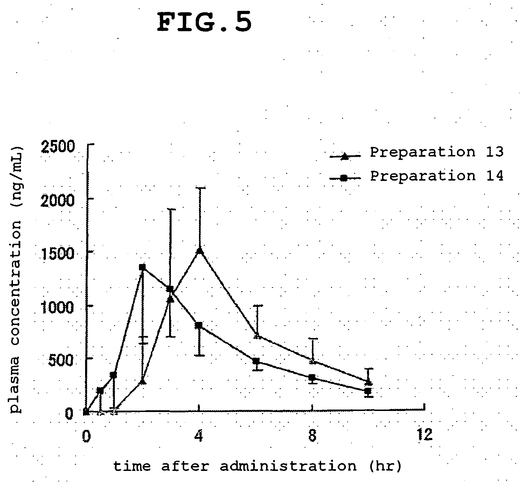

The coated tablets obtained in Examples 10 and 11 (Preparations 13 and 14) were orally administered to beagle dogs (male, body weight of about 10 kg), and the change in the plasma concentration of Compound A thereafter was investigated. The results are presented in Fig. 5.

As shown in Fig. 5, the blood concentration of the physiologically active ingredient (Compound A) was sustained by the controlled release composition of the invention.

### Experimental Example 6

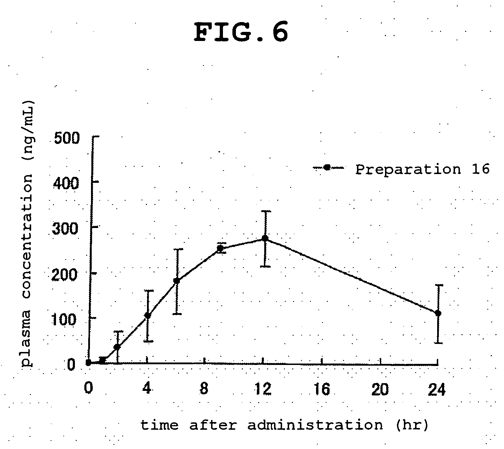

The capsules obtained in Example 13 (Preparation 16) was orally administered to cynomolgus monkeys (male, body weight of about 4 kg), and the change in the plasma concentration of Compound A thereafter was investigated. The results are presented in Fig. 6.

As shown in Fig. 6, the blood concentration of the physiologically active ingredient (Compound A) was sustained by the controlled release composition of the invention.

### Industrial Applicability

According to the present invention, a controlled release composition for oral administration of an imidazole derivative, which has steroid C_{17,20}-lyase inhibiting activity and which has remarkably improved sustainability of the blood concentration, is provided.

## Claims

1. A controlled release composition for oral administration, which comprises a physiologically active substance which is a compound represented by the formula: 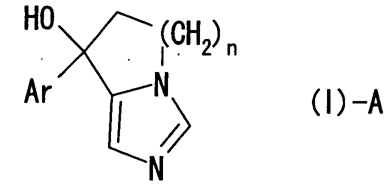 wherein n is an integer of 1 to 3, and Ar is an aromatic ring which may be substituted,
or a salt thereof, and a hydrophilic polymer.

2. A controlled release composition for oral administration, wherein a core containing a physiologically active substance which is a compound represented by the formula: 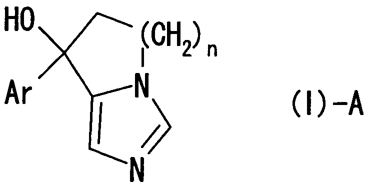 wherein n is an integer of 1 to 3, and Ar is an aromatic ring which may be substituted,or a salt thereof is coated with a coating layer containing a polymer.

3. The controlled release composition according to claim 1 or 2, wherein the solubility (37°C) of the physiologically active substance with respect to 1^{st} fluid for the disintegration test in the Japanese Pharmacopoeia is about 0.1 mg/mL or more.

4. The controlled release composition according to claim 1, which has the following dissolution characteristics:
1) in Method 2 of the dissolution test in the Japanese Pharmacopoeia (paddle method, rotation speed of paddle: 50 rpm, 37°C) using 900 mL of 1^{st} fluid for the disintegration test in the Japanese Pharmacopoeia, the dissolution rate of the physiologically active substance from the controlled release composition at 15 minutes after initiation of the test is less than 40%, and
2) in Method 2 of the dissolution test in the Japanese Pharmacopoeia (paddle method, rotation speed of paddle: 50 rpm, 37°C) using 900 mL of 2^{nd} fluid for the disintegration test in the Japanese Pharmacopoeia, the dissolution rate of the physiologically active substance from the controlled release composition at 24 hours after initiation of the test is 40% or more.

5. A controlled release composition for oral administration, which comprises
(1) a physiologically active substance which is a compound represented by the formula: 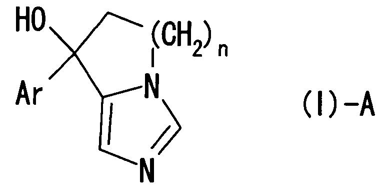 wherein n is an integer of 1 to 3, and Ar is an aromatic ring which may be substituted,
or a salt thereof,
(2) a hydrophilic polymer selected from hydroxypropylcellulose, hydroxypropylmethylcellulose, methylcellulose, polyethylene oxide, sodium carboxymethylcellulose and low-substituted hydroxypropylcellulose, and
(3) a lubricant selected from magnesium stearate, calcium stearate, talc, light anhydrous silicic acid, colloidal silica, synthetic aluminum silicate and magnesium aluminometasilicate.

6. The controlled release composition according to claim 2, which has the following dissolution characteristics:
1) in Method 2 of the dissolution test in the Japanese Pharmacopoeia (paddle method, rotation speed of paddle: 50 rpm, 37°C) using 900 mL of 1^{st} fluid for the disintegration test in the Japanese Pharmacopoeia, the dissolution rate of the physiologically active substance from the controlled release composition at 15 minutes after initiation of the test is less than 10%, and
2) in Method 2 of the dissolution test in the Japanese Pharmacopoeia (paddle method, rotation speed of paddle: 50 rpm, 37°C) using 900 mL of 2^{nd} fluid for the disintegration test in the Japanese Pharmacopoeia, the dissolution rate of the physiologically active substance from the controlled release composition at 24 hours after initiation of the test is 40% or more.

7. A controlled release composition for oral administration, wherein
(A) a core containing (1) a physiologically active substance which is a compound represented by the formula: 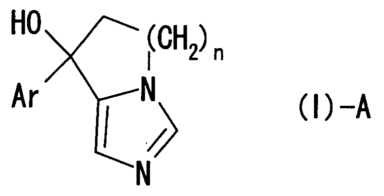 wherein n is an integer of 1 to 3, and Ar is an aromatic ring which may be substituted, or a salt thereof, and (2) hydrophilic polymers selected from hydroxypropylcellulose, hydroxypropylmethylcellulose, methylcellulose, polyethylene oxide, sodium carboxymethylcellulose and low-substituted hydroxypropylcellulose, is coated with
(B) a coating layer containing (1) a enteric coating agent selected from cellulose acetate phthalate, hydroxypropylmethylcellulose phthalate, hydroxymethylcellulose acetate succinate and methacrylic acid copolymers, (2) a lubricant selected from magnesium stearate, calcium stearate, talc, light anhydrous silicic acid, colloidal silica, synthetic aluminum silicate and magnesium aluminometasilicate, and (3) a plasticizer selected from acetyl tributyl citrate, acetyl triethyl citrate, castor oil, diacetylated monoglyceride, dibutyl sebacate, diethyl phthalate, glycerin, mono- and diacetylated monoglyceride, polyethylene glycol, propylene glycol, triacetin and triethyl citrate.

8. The controlled release composition according to claim 2, wherein the release property of the physiologically active substance in the absence of the coating layer is of rapid release.

9. The controlled release composition according to claim 2, wherein the core is a controlled release matrix which further comprises a hydrophilic polymer.

10. The controlled release composition according to claim 1 or 9, wherein the content of the hydrophilic polymer is about 3% to about 95% by weight.

11. The controlled release composition according to claim 2, wherein the polymer in the coating layer exhibits pH-dependent or delayed-dissolution type water solubility.

12. The controlled release composition according to claim 2, wherein the polymer in the coating layer is insoluble or sparingly soluble in water.

13. A controlled release composition, wherein the controlled release composition according to claim 1 or 2 is coated with a coating layer which contains a physiologically active substance which is identical with or different from the physiologically active substance contained in the above-mentioned controlled release composition, and the release property of the physiologically active substance being of rapid release.

14. The controlled release composition according to claim 1 or 2, which is used for prevention or treatment of prostate cancer or breast cancer.

15. A composition which comprises the controlled release composition according to claim 1 or 2, combined with at least one other controlled release composition wherein a release rate of a physiologically active substance is different from that of the above-mentioned controlled release composition.

16. The composition according to claim 15, wherein the other controlled release composition contains a physiologically active substance whose solubility (37°C) with respect to 1^{st} fluid for the disintegration test in the Japanese Pharmacopoeia is about 0.1 mg/mL or more.

17. The composition according to claim 16, wherein the physiologically active substance in the other controlled release composition is a compound represented by the formula: 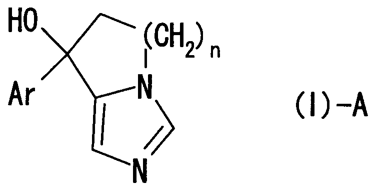 wherein n is an integer of 1 to 3, and Ar is an aromatic ring which may be substituted,
or a salt thereof.

18. The composition according to claim 17, which has the following dissolution characteristics:
1) in Method 2 of the dissolution test in the Japanese Pharmacopoeia (paddle method, rotation speed of paddle: 50 rpm, 37°C) using 900 mL of 1^{st} fluid for the disintegration test in the Japanese Pharmacopoeia, the dissolution rate of the physiologically active substance from the controlled release composition at 15 minutes after initiation of the test is less than 10%, and
2) in Method 2 of the dissolution test in the Japanese Pharmacopoeia (paddle method, rotation speed of paddle: 50 rpm, 37°C) using 900 mL of 2^{nd} fluid for the disintegration test in the Japanese Pharmacopoeia, the dissolution rate of the physiologically active substance from the controlled release composition at 24 hours after initiation of the test is 20% or more.

19. The composition according to claim 15, wherein the release property of the physiologically active substance in the other controlled release composition is of rapid release.

20. The composition according to claim 15, wherein the other controlled release composition is prepared by coating a core containing a physiologically active substance with a coating layer containing a polymer which exhibits pH-dependent or delayed-dissolution type water solubility.

21. The composition according to claim 15, which is used for prevention or treatment of prostate cancer or breast cancer.

22. A controlled release composition for oral administration, which comprises
(1) (+)-6-(7-hydroxy-6,7-dihydro-5H-pyrrolo[1,2-c]imidazol-7-yl)-N-methyl-2-naphthamide or a salt thereof,
(2) a hydrophilic polymer selected from hydroxypropylcellulose, hydroxypropylmethylcellulose, methylcellulose, polyethylene oxide, sodium carboxymethylcellulose and low-substituted hydroxypropylcellulose,
(3) a disintegrant selected from lactose, sucrose, starch, carboxymethylcellulose, calcium carboxymethylcellulose, sodium croscarmellose, sodium carboxymethyl starch, light anhydrous silicic acid and low-substituted hydroxypropylcellulose,
(4) a lubricant selected from magnesium stearate, calcium stearate, talc, light anhydrous silicic acid, colloidal silica, synthetic aluminum silicate and magnesium aluminometasilicate,
(5) a enteric coating agent selected from cellulose acetate phthalate, hydroxypropylmethylcellulose phthalate, hydroxymethylcellulose acetate succinate and methacrylic acid copolymers,
(6) a binder selected from α-starch, sugar, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose and polyvinylpyrrolidone, and
(7) a plasticizer selected from acetyl tributyl citrate, acetyl triethyl citrate, castor oil, diacetylated monoglyceride, dibutyl sebacate, diethyl phthalate, glycerin, mono- and diacetylated monoglyceride, polyethylene glycol, propylene glycol, triacetin and triethyl citrate.

23. A controlled release composition for oral administration, wherein
(A) a core containing (1) (+)-6-(7-hydroxy-6,7-dihydro-5H-pyrrolo[1,2-c]imidazol-7-yl)-N-methyl-2-naphthamide or a salt thereof, and (2) a hydrophilic polymer selected from hydroxypropylcellulose, hydroxypropylmethylcellulose, methylcellulose, polyethylene oxide, sodium carboxymethylcellulose and low-substituted hydroxypropylcellulose, is coated with
(B) a coating layer containing (1) an enteric coating agent selected from cellulose acetate phthalate, hydroxypropylmethylcellulose phthalate, hydroxymethylcellulose acetate succinate and methacrylic acid copolymers, (2) a lubricant selected from magnesium stearate, calcium stearate, talc, light anhydrous silicic acid, colloidal silica, synthetic aluminum silicate and magnesium aluminometasilicate, and (3) a plasticizer selected from acetyl tributyl citrate, acetyl triethyl citrate, castor oil, diacetylated monoglyceride, dibutyl sebacate, diethyl phthalate, glycerin, mono- and diacetylated monoglyceride, polyethylene glycol, propylene glycol, triacetin and triethyl citrate.
